# EUROPEAN PATENT APPLICATION

(11) **EP 2 891 464 A1**
(43) Date of publication of application: **08.07.2015**
(21) Application number: 13852731.2
(22) Date of filing: 01.11.2013
(51) Int. Cl.: A61B 18/12

(54) **THERAPEUTIC TOOL AND THERAPEUTIC METHOD**

(30) Priority: 09.11.2012 US 201261724532 P
(71) Applicant: OLYMPUS CORPORATION, Shibuya-ku Tokyo 151-0072 (JP)
(72) Inventor: TAKASHINO Tomoyuki, Tokyo 151-0072 (JP); SOBAJIMA Hideo, Tokyo 151-0072 (JP); TAKEI Yusuke, Tokyo 151-0072 (JP)
(74) Representative: Gunzelmann, Rainer
(86) International application number: PCT/JP2013/079745
(87) International publication number: WO 2014/073491

(57) **Abstract**

Provided is a medical treatment instrument including: a pair of openable/closable pinching portions that pinch a body tissue via a pair of pinching surfaces facing each other; an energy output section provided in at least one of the pair of pinching portions, the energy output section including an energy application surface for applying energy to the body tissue as at least one of the pair of pinching surfaces; a cutting member for cutting the body tissue that is capable of advancing/retracting inside the pinching portion; a guiding groove including a recess portion in the energy application surface, the guiding groove guiding the cutting member in such a manner that the cutting member can advance/retract inside the pinching portions; and gap maintaining portions arranged so as to be adjacent to a surface that is flush with an inner face of the recess portion of the guiding groove or be embedded in the guiding groove, the gap maintaining portions projecting between the pair of pinching surfaces facing each other when the pair of pinching portions are closed, to thereby maintain a fixed distance between the pair of pinching surfaces.

## Description

### Technical Field

The present invention relates to a medical treatment instrument and a medical treatment method, for applying energy to a body tissue and treating a body tissue.

### Background Art

Conventionally, a variety of medical treatment instruments and medical treatment methods for applying energy to a body tissue and treating the body tissue have been proposed.

Here, examples of energy to be applied to, for example, bond body tissues to join the tissues include high-frequency energy and resistance heat energy. For example, when high-frequency energy is applied from electrodes, in order to prevent shorting of electrodes, gap maintaining means for defining a minimum interval between the respective electrodes is provided.

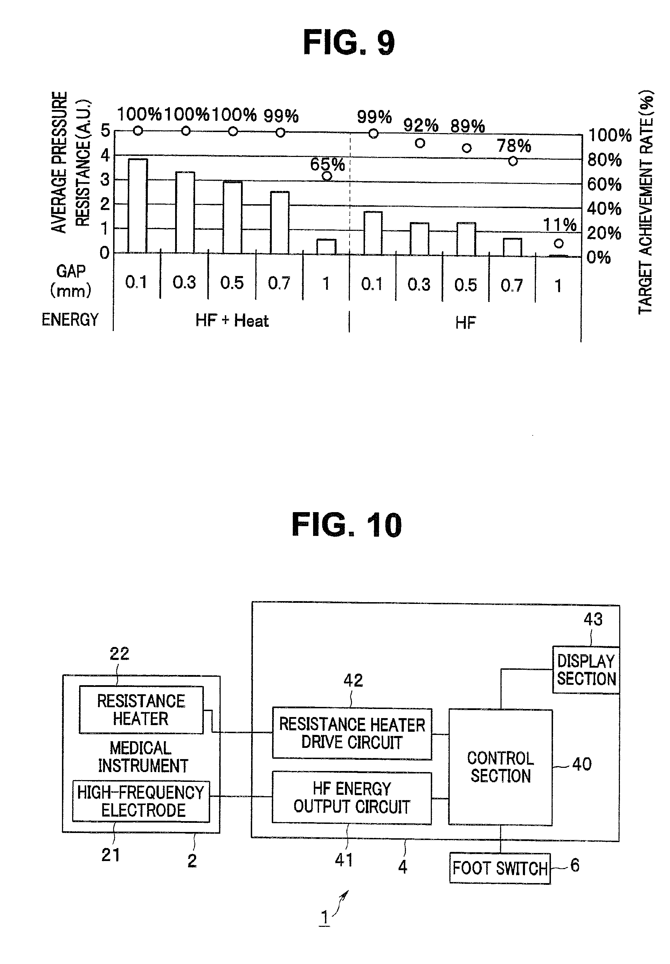

For example, paragraph [0062] and Figs. 9, 20 and 24B, etc., of U.S. Patent Application Publication No. 2005/0154387A1 describe at least one stop member 175 being disposed on, e.g., an electrically conductive sealing surface 122, the stop member 175 preferably defining a distance between jaws during sealing as a gap distance G, the gap distance G being within the range of about 0.03 mm to around 0.016 mm, and also a stop member 175 being preferably positioned on either side of a knife channel 115 (however, as can be seen from Fig. 9, a stop member 175 is not only provided at a position close to the knife channel 115, but also at a distal end portion of the electrically conductive sealing surface 122, which is distant from knife channel 115).

Also, paragraph [0106] and Fig. 23 of U.S. Patent Application Publication No. 2007/0078458A1 include description and illustration similar to those of U.S. Patent Application Publication No. 2005/0154387A1.

Furthermore, paragraphs [0048] and [0060] and Figs. 5 and 2, etc., of U.S. Patent Application Publication No. 2008/0071268A1 describe electrode parts 22 and 23 including open regions 22d and 23d that guide a cutting member 30 including a blade 31, and a protruding insulating section 28 for preventing shorting of the electrode parts 22 and 23 being formed from two parts 28a and 28a' and being provided directly adjacent to an open region 22d. It is also described that these two parts 28a and 28a' extend preferably parallel to crests of the electrode parts 22 and 23.

Also, paragraph [0069] and Fig. 12 of U.S. Patent Application Publication No. 2002/0198525A1 describe a follower 47 of an upper jaw 42 abutting a cam 45 of a lower jaw 44 so that the upper jaw 42 fits tightly against the lower jaw 44 with very minimum air gaps therebetween.

From the perspective of prevention of shorting of the electrodes, the aforementioned gap maintaining means is formed by a non-electrically conductive material, which means that in parts where the gap maintaining means are provided, high-frequency energy is not applied to a tissue.

However, when treatment for sealing a tissue, for example, is performed, it is preferable to seal the tissue in a largest possible continuous area.

Therefore, it is preferable to enable medical treatment with a higher treatment effect to be performed, while preventing shorting of an energy application surface.

The present invention has been achieved in view of the above-described circumstances, and an object of the present invention is to provide a medical treatment instrument and a medical treatment method that enable medical treatment with a higher treatment effect to be performed, while preventing shorting of an energy application surface.

### Disclosure of Invention

### Means for Solving the Problem

A medical treatment instrument according to one aspect of the present invention is a medical treatment instrument for applying energy to a body tissue and treating the body tissue, the medical treatment instrument including: a pair of openable/closable pinching portions that pinch the body tissue via a pair of pinching surfaces facing each other; an energy output section provided in at least one of the pair of pinching portions, the energy output section including an energy application surface for applying energy to the body tissue as at least one of the pair of pinching surfaces; a cutting member for cutting off the body tissue pinched between the pinching portions, the cutting member being capable of advancing/retracting inside the pinching portions; a guiding groove including a recess portion in the energy application surface, the guiding groove guiding the cutting member in such a manner that the cutting member can advance/retract inside the pinching portions; and gap maintaining portions arranged so as to be adjacent to a surface that is flush with an inner face of the recess portion of the guiding groove or be embedded in the guiding groove, the gap maintaining portions projecting between the pair of pinching surfaces facing each other when the pair of pinching portions are closed, to thereby maintain a fixed distance between the pair of pinching surfaces.

In addition, a medical treatment method according to another aspect of the present invention is a medical treatment method for applying energy to a body tissue and treating the body tissue by using a medical treatment instrument including: a pair of openable/closable pinching portions that pinch the body tissue via a pair of pinching surfaces facing each other; an energy output section provided in at least one of the pair of pinching portions, the energy output section including an energy application surface for applying energy to the body tissue as at least one of the pair of pinching surfaces; a cutting member for cutting off the body tissue pinched between the pinching portions, the cutting member being capable of advancing/retracting inside the pinching portions; a guiding groove including a recess portion in the energy application surface, the guiding groove guiding the cutting member in such a manner that the cutting member can advance/retract inside the pinching portions; and gap maintaining portions arranged so as to be adjacent to a surface that is flush with an inner face of the recess portion of the guiding groove or be embedded in the guiding groove, the gap maintaining portions projecting between the pair of pinching surfaces facing each other when the pair of pinching portions are closed, to thereby maintain a fixed distance between the pair of pinching surfaces, the medical treatment method including: pinching the body tissue via the pair of pinching portions by maintaining a distance between the pair of pinching surfaces to the fixed distance by the gap maintaining portions when the pair of pinching portions are closed; applying energy to the body tissue from the energy application surface to treat the body tissue while pinching the body tissue via the pair of pinching portions; and cutting a part of the body tissue adjacent to the gap maintaining portions by advancing the cutting member along the guiding groove.

### Brief Description of the Drawings

Fig. 1 is a perspective view illustrating a configuration of a medical treatment system including a linear medical treatment instrument according to a first embodiment of the present invention;
Fig. 2 is a vertical cross-sectional view of a shaft, and a pinching portion in a closed state, of the medical treatment instrument according to the first embodiment;
Fig. 3 is a vertical cross-sectional view of the shaft, and the pinching portion in an opened state, of the medical treatment instrument according to the first embodiment;
Fig. 4 is a plan view illustrating a first pinching member in the medical treatment instrument according to the first embodiment;
Fig. 5 is a cross-sectional view of the first pinching member in the medical treatment instrument according to the first embodiment, along line 5-5 in Fig. 4;
Fig. 6 is a perspective view illustrating a back face of a first high-frequency electrode with resistance heaters provided thereon in the first embodiment;
Fig. 7 is a perspective view illustrating another example configuration of a resistance heater provided on the back face of the first high-frequency electrode in the first embodiment;
Fig. 8 is a graph indicating an example pressure resistance performance and target achievement rate test of sealed body tissues obtained by applying energy to first tissue samples with a gap varied in the first embodiment;
Fig. 9 is a graph indicating an example pressure resistance performance and target achievement rate test of sealed body tissues obtained by applying energy to second tissue samples with a gap varied in the first embodiment;
Fig. 10 is a block diagram illustrating mainly an electrical configuration of a medical treatment system in the first embodiment;
Fig. 11 is a flowchart illustrating processing for providing treatment using high-frequency energy and heat energy to a body tissue, using the medical treatment system of the first embodiment;
Fig. 12 is a plan view illustrating a first pinching member of a medical treatment instrument according to a first modification of the first embodiment;
Fig. 13 is a cross-sectional view of the first pinching member of the medical treatment instrument according to the first modification of the first embodiment, along line 13-13 in Fig. 12;
Fig. 14 is a plan view illustrating a first pinching member of a medical treatment instrument according to a second modification of the first embodiment;
Fig. 15 is a plan view of a first pinching member of a medical treatment instrument according to a third modification of the first embodiment;
Fig. 16 is a cross-sectional view of the first pinching member of the medical treatment instrument according to the third modification of the first embodiment, along line 16-16 in Fig. 15;
Fig. 17 is a plan view illustrating a first pinching member of a medical treatment instrument according to a fourth modification of the first embodiment;
Fig. 18 is a cross-sectional view of the first pinching member of the medical treatment instrument according to the fourth modification of the first embodiment, along line 18-18 in Fig. 17;
Fig. 19 is a plan view illustrating a first pinching member of a medical treatment instrument according to a fifth modification of the first embodiment;
Fig. 20 is a side view illustrating a configuration of a pinching portion configured as a seesaw-type one in a medical treatment instrument according to a second embodiment of the present invention;
Fig. 21 is a perspective view illustrating a configuration of a medical treatment system including a circular medical treatment instrument according to a third embodiment of the present invention;
Fig. 22 is a vertical cross-sectional view of the medical treatment instrument according to the third embodiment when a detachable-side pinching portion is separated from a body-side pinching portion; and
Fig. 23 is a plan view illustrating a configuration of a pinching surface of the body-side pinching portion of the medical treatment instrument according to the third embodiment.

### Best Mode for Carrying Out the Invention

Embodiments of the present invention will be described below with reference to the drawings.

### [First Embodiment]

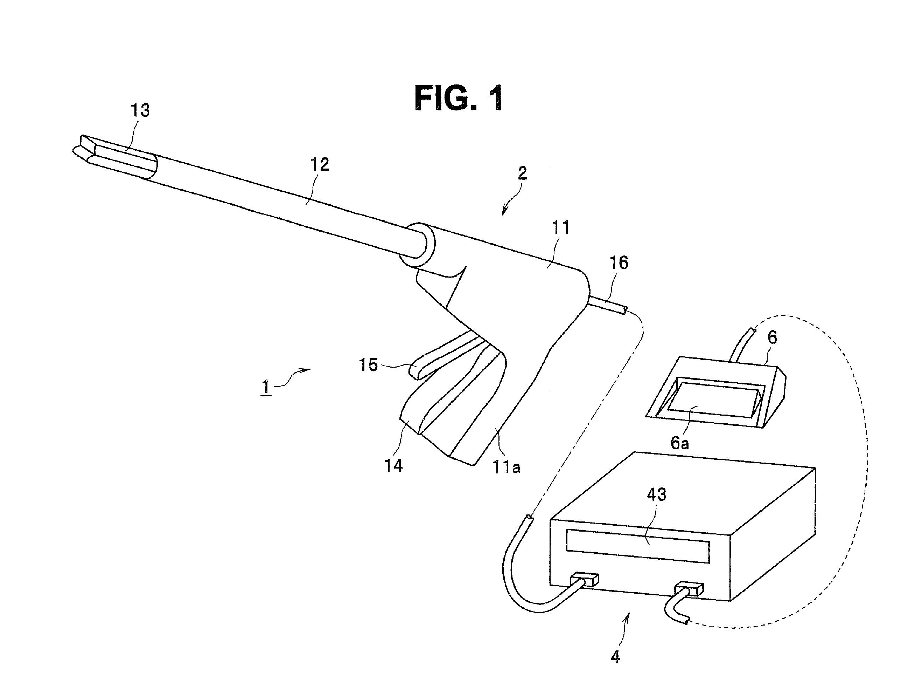

Figs. 1 to 19 illustrate a first embodiment of the present invention, and Fig. 1 is a perspective view illustrating a configuration of a medical treatment system including a linear medical treatment instrument.

The present embodiment is an embodiment in which a medical treatment instrument (energy treatment instrument) is a linear medical treatment instrument 2 for providing treatment through, for example, an abdominal wall. However, the medical treatment instrument may be a linear medical treatment instrument for open surgery, which is performed by picking out a treatment target body tissue through an abdominal wall for treatment.

As illustrated in Fig. 1, a medical treatment system 1 includes the medical treatment instrument 2, an energy source 4 and a foot switch 6.

The medical treatment instrument 2 includes a handle 11, an elongated shaft 12 provided so as to extend from the distal end side of the handle 11, and an openable/closable pinching portion 13 disposed on the distal end side of the shaft 12.

The handle 11 is connected via a cable 16 to the energy source 4 including a display section 43. The foot switch 6 including a pedal 6a (however, the switch 6 is not limited to a foot switch and for example, may be a hand switch) is connected to the energy source 4. Upon a surgeon operating the pedal 6a of the foot switch 6, supply of energy from energy source 4 to the medical treatment instrument 2 is switched on or off.

The handle 11 is formed so as to have a shape that a surgeon can easily grasp, for example, a rough L shape. The shaft 12 is disposed at an end of the handle 11. The aforementioned cable 16 extends from a proximal end of the handle 11, which is coaxial with the shaft 12. Also, another end side of the handle 11 provides a grasping portion 11a to be held by a surgeon with his/her hand.

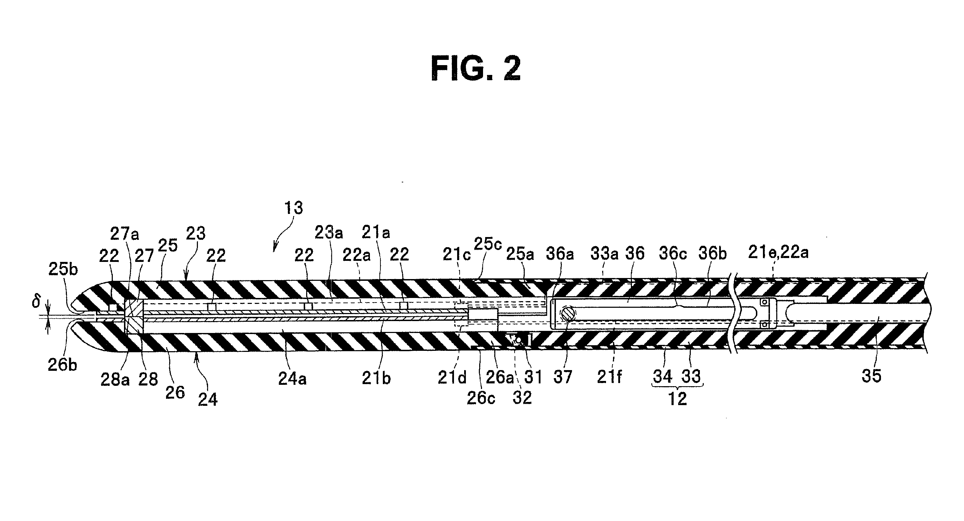

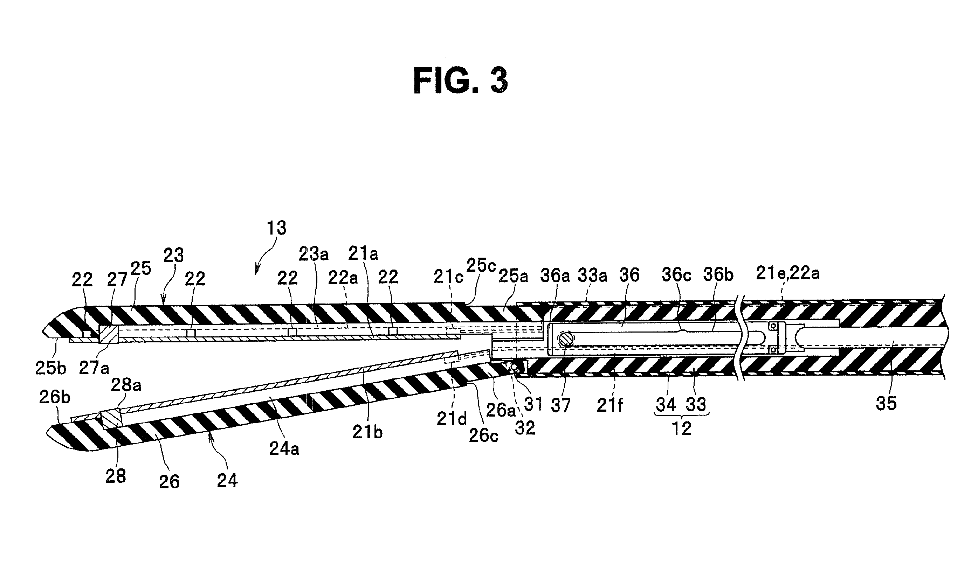

The handle 11 includes a pinching portion opening/closing lever 14 is pivotably provided in such a manner that the pinching portion opening/closing lever 14 is arranged side by side with the grasping portion 11a. The pinching portion opening/closing lever 14 is joined to a proximal end portion of a later-described sheath 34 (see Figs. 2 and 3), which form an outer envelope of the shaft 12, at a rough central part of the handle 11. Upon the pinching portion opening/closing lever 14 being operated so as to move close to or away from the grasping portion 11a of the handle 11, the sheath 34 moves along an axial direction of the shaft 12. If the sheath 34 moves toward the distal end side in the axial direction of the shaft 12, the pinching portion 13 is closed, and if the sheath 34 moves toward the proximal end side in the axial direction, the pinching portion 13 is opened.

The handle 11 also includes a cutter drive lever 15 pivotably provided in such a manner that the cutter drive lever 15 is arranged side by side with the pinching portion opening/closing lever 14. Upon the cutter drive lever 15 being operated to move close to or away from the grasping portion 11a of the handle 11, a later-described cutter 36 (see Figs. 2 and 3), which is a cutting member, moves along the axial direction of the shaft 12 to cut a body tissue off.

Fig. 2 is a vertical cross-sectional view illustrating the shaft, and the pinching portion in a closed state, of the medical treatment instrument, and Fig. 3 is a vertical cross-sectional view of the shaft, and the pinching portion in an opened state, of the medical treatment instrument.

As illustrated in Figs. 2 and 3, the shaft 12 includes a tubular body 33 having, for example, a rough cylindrical shape, and the sheath 34 having a thin cylindrical shape, the sheath 34 being disposed so as to be slidable relative to an outer circumference of the tubular body 33. The tubular body 33 is fixed to the handle 11 (see Fig. 1) at a proximal end portion thereof. The sheath 34 is slidable along an axial direction of the tubular body 33.

In the outer circumference of the tubular body 33, a groove 33a formed along the axial direction of the tubular body 33 is formed. In the groove 33a, a first high-frequency electrode energization line 21 e, and a heater energization line 22a are disposed. The first high-frequency electrode energization line 21e is connected to a later-described first high-frequency electrode 21 a, and the heater energization line 22a is connected to later-described resistance heaters 22.

Also, inside the tubular body 33, a second high-frequency electrode energization line 21f is inserted. The second high-frequency electrode energization line 21f is connected to a later-described second high-frequency electrode 21b.

Furthermore, inside the tubular body 33, a drive rod 35 having, for example, a columnar shape is disposed so as to be movable along an axial direction thereof. A thin plate-like cutter 36, which is a cutting member that serves as a treatment aid, is fixed to a distal end portion of the drive rod 35. Also, a proximal end portion of the drive rod 35 is joined to the cutter drive lever 15. Therefore, upon the cutter drive lever 15 being operated, the cutter 36 moves in the axial direction via the drive rod 35.

At a distal end of the cutter 36, a blade 36a for cutting a body tissue off is formed. A long hole 36b, which serves as an axial guide hole, is formed between the distal end and a proximal end of the cutter 36. A movement restricting pin 37 engages the long hole 36b. The movement restricting pin 37 is fixed to the tubular body 33 in such a manner that the movement restricting pin 37 extends in a direction perpendicular to the axial direction of the shaft 12. Thus, the cutter 36 linearly moves in the axial direction while the state in which the long hole 36b engages with the movement restricting pin 37 is maintained. Then, upon the cutter 36 moving in the distal end direction, the cutter 36 enters cutter guiding grooves 23a and 24a of a first pinching member 23 and a second pinching member 24, which will be described later.

Here, in each of at least three positions, i.e., an end, the other end and a position between the one end and the other end of the long hole 36b of the cutter 36, a locking portion 36c that locks the movement restricting pin 37 to control movement of the cutter 36 is formed.

As illustrated in Figs. 2 and 3, the pinching portion 13 has a shape extending in a longitudinal direction from a proximal end portion toward a distal end portion, and is configured in such a manner that the first pinching member 23, which is also called first jaw, and the second pinching member 24, which is also called second jaw, are joined via a pivot shaft on the proximal end side and at least one of the pinching members (in the present embodiment, the second pinching member 24 as illustrated in Fig. 3) is pivotable about the pivot shaft.

The first pinching member 23 and the second pinching member 24 themselves preferably have electrical insulating properties in their entireties except electrical circuit parts such as electrodes, heaters and energization lines. Therefore, a first pinching member body 25 included in the first pinching member 23 and a second pinching member body 26 included in the second pinching member 24 are formed by an electrical insulating material.

A base portion 25a, which is a proximal end portion of the first pinching member body 25, is fixed to a distal end portion of the tubular body 33 of the shaft 12. On the other hand, a base portion 26a, which is a proximal end portion of the second pinching member body 26, is pivotably supported on the distal end portion of the tubular body 33 of the shaft 12 via a support pin 31 disposed in the direction perpendicular to the axial direction of the shaft 12. Accordingly, the second pinching member 24 can be opened/closed relative to the first pinching member 23 by making the second pinching member 24 pivot about the support pin 31. Furthermore, the second pinching member 24 is biased by an elastic member 32 such as a plate spring in a direction in which the second pinching member 24 is opened relative to the first pinching member 23.

The first pinching member body 25 and the second pinching member body 26 have respective outer surfaces formed so that when the first pinching member body 25 and the second pinching member body 26 are closed, a cross section of the member bodies put together has a smooth curved shape such as a rough round shape or a rough oval shape. Although the base portions 25a and 26a also have respective outer surfaces formed to provide a smooth round shape, the base portions 25a and 26a are configured so as to have a diameter somewhat smaller than that of the distal end side, whereby respective steps 25c and 26c are formed between the base portions 25a and 26a and the distal end side. More specifically, the diameters of the base portions 25a and 26a when the first pinching member body 25 and the second pinching member body 26 are closed are substantially equal to or slightly smaller than an inner diameter of the sheath 34 (is substantially equal to or slightly larger than a diameter of the tubular body 33 of the shaft 12), and diameters of the first and second pinching member bodies 25 and 26 are larger than the inner diameter of the sheath 34. Therefore, if the sheath 34 is made to slide relative to the tubular body 33, the sheath 34 can advance to a position where the sheath 34 covers the base portions 25a and 26a, but the advancement is blocked at positions of the steps 25c and 26c.

With such configuration, if the sheath 34 is made to slide toward the distal end side against a biasing force of the elastic member 32, as illustrated in Fig. 2, the first pinching member 23 and the second pinching member 24 are closed. On the other hand, if the sheath 34 is made to slide on the proximal end side from a state in which the base portions 25a and 26a are covered by the distal end portion of the sheath 34, as illustrated in Fig. 3, the second pinching member 24 is opened relative to the first pinching member 23 via the biasing force of the elastic member 32.

The first pinching member body 25 includes the cutter guiding groove 23a including a recess portion in an energy application surface and the second pinching member body 26 includes the cutter guiding groove 24a having a recess portion in an energy application surface, which are formed in such a manner that when the pinching portion 13 is closed, the cutter guiding groove 23a and the cutter guiding groove 24a face each other and extend in a direction along the axial direction of the shaft 12. The cutter guiding grooves 23a and 24a are structure portions for guiding the above-described cutter 36 to the inside of the pinching portion 13 from respective proximal end portions to respective distal end portions along a center axis in the longitudinal direction, and in a state in which the pinching portion 13 is closed, the cutter 36 advances/retracts inside a hole formed by putting the two cutter guiding grooves 23a and 24a together. Each of the distal end sides of two cutter guiding grooves 23a and 24a terminates inside the pinching portion 13, that is, none of the distal end sides is communicated with the outside. Therefore, even when the cutter 36 advances furthest in the distal end direction, the cutter 36 remains inside the pinching portion 13.

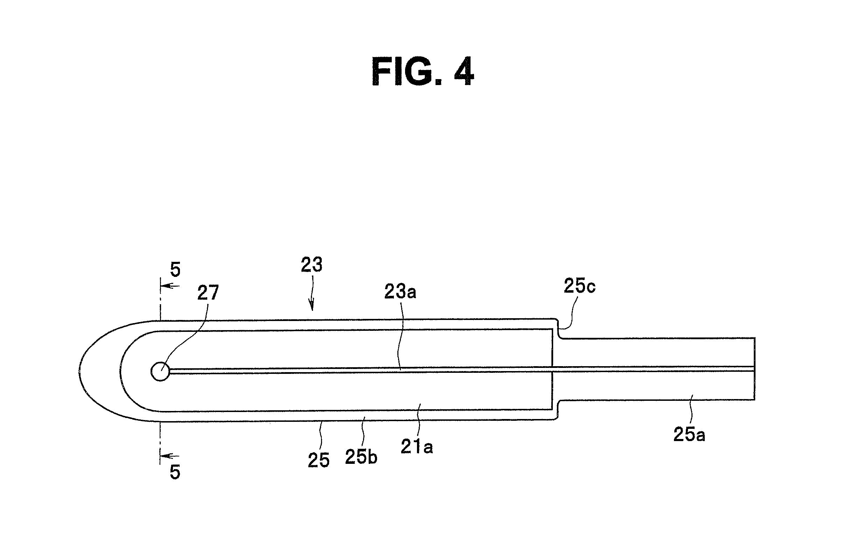

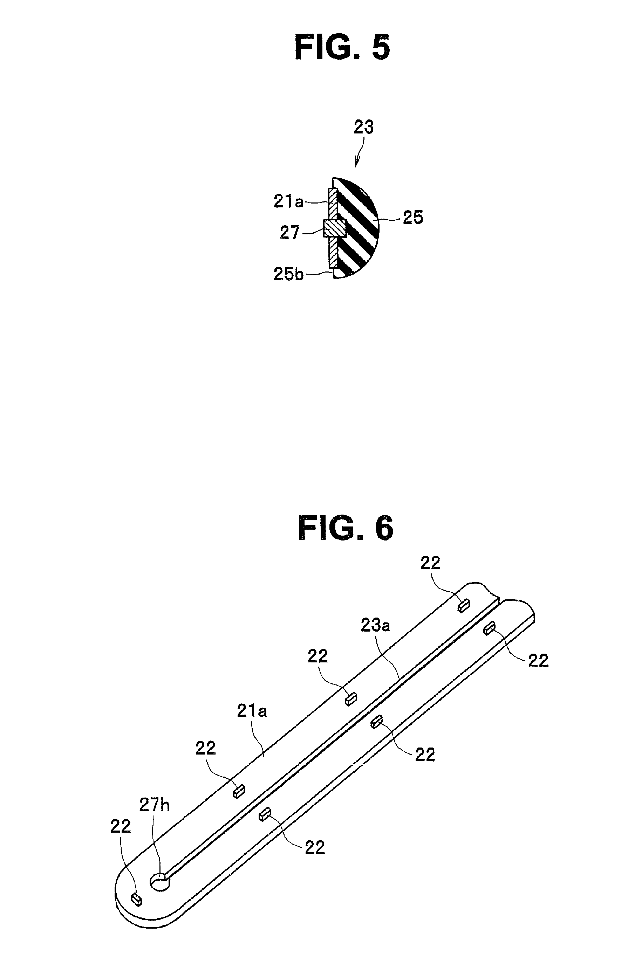

Fig. 4 is a plan view illustrating the first pinching member of the medical treatment instrument, and Fig. 5 is a cross-sectional view of the first pinching member of the medical treatment instrument along line 5-5 in Fig. 4.

As illustrated in Figs. 2 to 5, the first pinching member body 25 includes a holding surface 25b, and on the holding surface 25b, the first high-frequency electrode 21a is disposed as an energy output section for applying high-frequency energy to a body tissue. Also, as illustrated in Figs. 2 and 3, the second pinching member body 26 includes a holding surface 26b, and on the holding surface 26b, the second high-frequency electrode 21b is disposed as an energy output section for applying high-frequency energy to a body tissue. Each of the first high-frequency electrode 21a and the second high-frequency electrode 21b is a member formed of an electrically conductive material.

As illustrated in Fig. 4, the first high-frequency electrode 21 a is formed in the shape of a flat plate having a size that allows the first high-frequency electrode 21 a to remain on the inner circumferential side relative to an edge of the holding surface 25b, for example, a rough U shaped flat plate, as described above, because the cutter guiding groove 23 a extending along the axial direction of the first pinching member 23 is formed so as to terminate inside the pinching portion 13.

The second high-frequency electrode 21b is also formed in the shape of a rough U shaped flat plate because the cutter guiding groove 24a terminates inside the pinching portion 13, which is substantially similar to the first high-frequency electrode 21 a illustrated in Fig. 4.

At a terminal end on the distal end side inside the cutter guiding groove 23a of the first pinching member body 25, a gap maintaining portion 27 formed of an electrical insulating member (non-electrically conductive material) is provided, and at a terminal end on the distal end side inside the cutter guiding groove 24a of the second pinching member body 26, a gap maintaining portion 28 formed of an electrical insulating member is provided, respectively. The gap maintaining portions 27 and 28 are provided upright in such a manner that the gap maintaining portions 27 and 28 are embedded in the cutter guiding grooves 23a and 24a, respectively, and project from a pair of pinching surfaces (the energy application surface of the first high-frequency electrode 21 a and the energy application surface of the second high-frequency electrode 21b), respectively. Here, specific examples of the electrical insulating material forming the gap maintaining portions 27 and 28 include, e.g., metals processed to have non-electrical conductivity, non-electrically conductive ceramics and non-electrically conductive resins, and each of the gap maintaining portions 27 and 28 is formed by at least one of these materials. Therefore, none of the gap maintaining portions 27 and 28 contributes to application of high-frequency energy, which is electrical energy, even if the gap maintaining portion 27 or 28 abuts a body tissue.

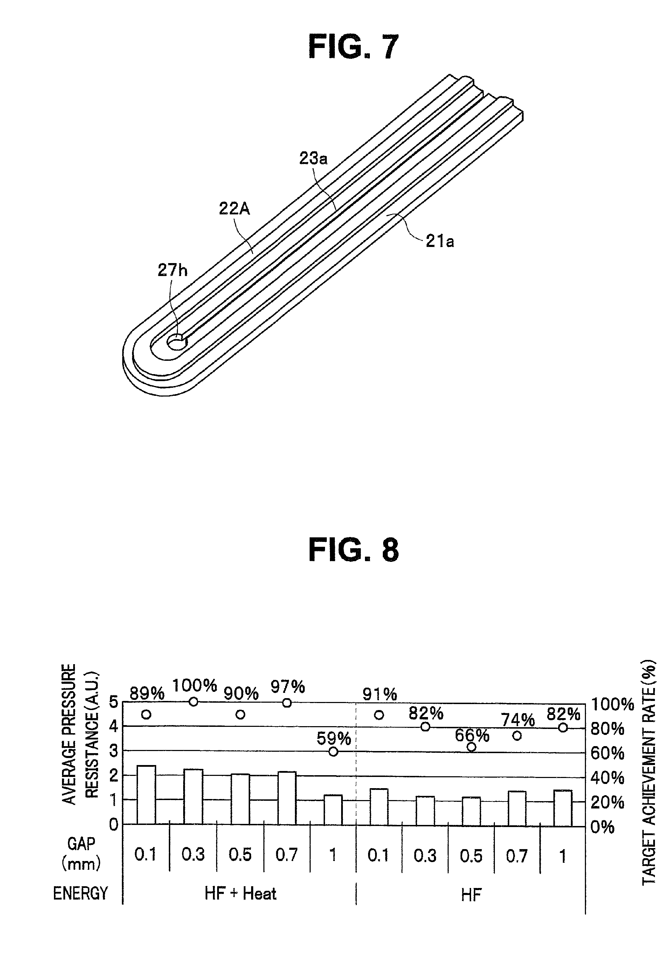

Also, a hole 27h for insertion of the gap maintaining portion 27 is provided in the first high-frequency electrode 21 a as illustrated in Fig. 6 (or Fig. 7 relating to a modification), which will be described later. Although not illustrated, likewise, a hole for insertion of the gap maintaining portion 28 is provided also in the second high-frequency electrode 21b.

Although in the illustrated example, the gap maintaining portions 27 and 28 have respective diameters that are larger than respective groove widths of the cutter guiding grooves 23a and 24a, it should be understood that the diameters may be made equal to the respective groove widths. However, where the gap maintaining portions 27 and 28 are ones intended to maintain a distance between the pair of pinching surfaces facing each other (the energy application surface of the first high-frequency electrode 21a and the energy application surface of the second high-frequency electrode 21b) at a fixed distance in one site such as illustrated in, e.g., Figs. 2, 3 and 5, the gap maintaining portions 27 and 28 preferably have larger diameters for stability and reliability enhancement.

The gap maintaining portions 27 and 28 are provided to, when the first pinching member 23 and the second pinching member 24 are closed, maintain the electrode surface-to-electrode surface distance between the energy application surface of the first high-frequency electrode 21 a and the energy application surface of the second high-frequency electrode 21b, which form a pair of pinching surfaces facing each other, at a fixed distance δ (see Fig. 2). In other words, in the example illustrated in Figs. 2 to 5, each of the gap maintaining portions 27 and 28 is formed as a boss (however, it should be understood that the shape of the gap maintaining portions 27 and 28 is not limited to a boss shape). Then, when the first pinching member 23 and the second pinching member 24 are closed, a head portion 27a of the gap maintaining portion 27 and a head portion 28a of the gap maintaining portion 28 butt each other, whereby the aforementioned fixed distance δ is maintained. In other words, the configuration is provided in such a manner that a total height of a height of the projection of the gap maintaining portion 27 from the energy application surface of the first high-frequency electrode 21 a and a height of the projection of the gap maintaining portion 28 from the energy application surface of the second high-frequency electrode 21b is the fixed distance δ. Here, as will be described later with reference to Figs. 8 and 9, the fixed distance δ is a distance of no more than 0.7 mm and furthermore, for example, a distance of no less than 0.2 mm. Then, in the closed state, the pair of pinching surfaces facing each other are, for example, parallel to each other.

However, the fixed distance δ is maintained where nothing or a tissue or the like having a thickness that is equal to or smaller than the distance δ is held between the pair of pinching surfaces. Therefore, if cases where a tissue that is thicker than the distance δ is held between the pair of pinching surfaces are contemplated, the gap maintaining portions 27 and 28 can be considered as ones intended to maintain the distance between the pair of pinching surfaces at the fixed distance δ or more.

As described above, the gap maintaining portions 27 and 28 are formed of an electrical insulating material and thus do not contribute to application of high-frequency energy, and thus, if each of the gap maintaining portions 27 and 28 is formed at a position partway on the energy application surface of the first high-frequency electrode 21 a or the second high-frequency electrode 21b (position that is not a peripheral edge), a discontinuous part in the form of an isolated island is formed on the energy application surface for application of energy to a body tissue. If such discontinuous parts in the form of isolated islands appear in a chain of islands or in such a manner that the discontinuous parts are linearly joined to each other inside a bonded tissue (inner portion, not a periphery of the tissue), such parts form a weak part of the bonded tissue, which causes decrease in pressure resistance performance (see also, e.g., Figs. 8 and 9).

On the other hand, the above-described configuration of the present embodiment, that is, the configuration in which the gap maintaining portions 27 and 28 are embedded in the respective terminal ends on the distal end side of the cutter guiding grooves 23 a and 24a enables maintenance of continuity of the respective energy application surfaces. Accordingly, when body tissues are bonded to each other, high pressure resistance performance can be maintained without formation of weak parts.

Furthermore, as illustrated in Figs. 2, 3 and 6, on the back face side of the first high-frequency electrode 21 a, resistance heaters 22 are disposed as an energy output section that generates resistance heat energy. Fig. 6 is a perspective view illustrating the back face of the first high-frequency electrode with the resistance heaters provided thereon.

In the example illustrated in Figs. 2, 3 and 6, the resistance heaters 22 have a chip-like shape, and are discretely disposed on the back face of the first high-frequency electrode 21 a along the rough U shape of the first high-frequency electrode 21 a. However, the first high-frequency electrode 21 a and the resistance heaters 22 are electrically insulated from each other. When the resistance heaters 22 are caused to produce heat, the heat is conducted to the first high-frequency electrode 21 a, via which the heat is delivered to a body tissue.

In such configuration, in order to conduct heat generated from the resistance heaters 22 to the first high-frequency electrode 21 a with small loss, the first pinching member body 25 preferably has not only electrical insulating properties as described above, but also heat insulating properties, and covers outer peripheries of the resistance heaters 22.

Also, Fig. 7 is a perspective view illustrating another example configuration of a resistance heater provided on the back face of the first high-frequency electrode 21a.

A resistance heater 22A, which is illustrated in Fig. 7, is an energy output section formed in a U shape along the shape of the first high-frequency electrode 21a, which forms a rough U shape. Here, an edge on the outer periphery side of the U-shaped resistance heater 22A remains inside an edge of the outer periphery side of the U-shaped first high-frequency electrode 21a without protruding from the edge of the first high-frequency electrode 21a, and an edge on the inner periphery side of the U-shaped resistance heater 22A remains inside an edge on the inner periphery side of the U-shaped first high-frequency electrode 21a without protruding from the edge of the first high-frequency electrode 21 a.

The resistance heater 22A is formed as, for example, a screen-printed thick film heat generating resistor, a thin film heat generating resistor formed by physical vapor deposition (PVD), an installed nichrome wire or any of other heat generators, on the back face of the first high-frequency electrode 21 a.

As illustrated in Figs. 2 and 3, the first and second high-frequency electrodes 21a and 21 b are connected to the first and second high-frequency electrode energization lines 21 e and 21 f, respectively, via the first and second electrode connectors 21c and 21d provided at respective proximal end portions, and further connected to the energy source 4 via the cable 16. Also, the resistance heaters 22 are connected to the heater energization line 22a and further connected to the energy source 4 via the cable 16.

As described above, the surfaces of the first high-frequency electrode 21 a and the second high-frequency electrode 21 b that face each other serve as pinching surfaces for coming into contact with and pinching a body tissue as well as energy application surfaces for applying high-frequency energy and resistance heat energy to the body tissue.

Next, an example test with a variety of gaps (fixed distance δ) between the aforementioned pair of pinching surfaces employed will be described with reference to Figs. 8 and 9. Fig. 8 is a graph indicating an example pressure resistance performance and target achievement rate test of sealed body tissues obtained by applying energy to first tissue samples with a gap varied, and Fig. 9 a graph indicating an example pressure resistance performance and target achievement rate test of sealed body tissues obtained by applying energy to second tissue samples with a gap varied. Here, the first tissue samples and the second tissue samples are samples obtained from different sites of a body tissue.

Figs. 8 and 9 indicate results of cases where high-frequency energy (HF) and resistance heat energy (Heat) were applied in the left half and results of cases where only high-frequency energy (HF) was applied in the right half, and the abscissa axis represents the size of the gap in millimeters.

Also, in Figs. 8 and 9, the ordinate axis represents an average pressure resistance of each sealed tissue in arbitrary unit (A.U.), and a target achievement rate indicating the degree of achievement of a target pressure resistance in percentage (%).

Where only high-frequency energy (HF) is applied to a first tissue sample as indicated in the right half of Fig. 8 to treat the first tissue sample, the average pressure resistance of the sealed tissue is between approximately 1.2 A.U. to approximately 1.5 A.U. regardless of employment of any of gaps of 0.1 to 1 mm, and thus, no dependency on the gap can be seen.

Also, the target achievement rate increases relative to a minimum rate of 66% when the gap is 0.5 mm, regardless of whether the gap is larger or smaller than that gap, the target achievement rate is 82% in each of the cases where the gap is 0.3 mm and where the gap is 1 mm. However, it can be seen that a high target achievement rate of no less than 80% can be obtained where the gap is no more than 0.3 mm.

On the other hand, where only high-frequency energy (HF) is applied to a second tissue sample as indicated in the right half of Fig. 9 to treat the second tissue sample, while the average pressure resistance of the sealed tissue is no less than approximately 1.3 A.U. if the gap is no more than 0.5 mm, the average pressure resistance decreases to approximately 0.8 A.U. if the gap is 0.7 mm, and extremely decreases to around 0.1 A.U. if the gap is 1 mm.

Also, the target achievement rate gradually decreases relative to a maximum value of 99% where the gap is 0.1 mm, and gradually decreases if the gap is larger than that gap, and extremely decreases to 11% if the gap is 1 mm.

Therefore, in summation of Figs. 8 and 9, it can be seen that if treatment is provided to plural kinds of tissues by means of application of high-frequency energy (HF) only, the gap is preferably no more than 0.3 mm to obtain a target achievement rate of no less than 80%.

Next, where high-frequency energy (HF) and resistance heat energy (Heat) are applied to a first tissue sample as indicated in the left half of Fig. 8 to treat the first tissue sample, while an average pressure resistance of no less than 2 A.U. can be obtained as the average pressure resistance of the sealed tissue if the gap is no more than 0.7 mm, the average pressure resistance decreases to around 1.2 A.U. if the gap is 1 mm.

Also, while a target achievement rate of at little less than 90% (89%) or more can be obtained if the gap is no more than 0.7 mm, the target achievement rate sharply decreases to 59% if the gap is 1 mm.

On the other hand, where high-frequency energy (HF) and resistance heat energy (Heat) are applied to a second tissue sample to treat the second tissue as indicated in the left half of Fig. 9, while an average pressure resistance of no less than approximately 2.5 A.U. can be obtained as the average pressure resistance of the tissue if the gap is no more than 0.7 mm, the average pressure resistance substantially decreases to around 0.6 A.U. if the gap is 1 mm.

Also, while a target achievement rate of no less than 99% can be obtained if the gap is no more than 0.7 mm, the target achievement rate extremely decreases to 65% if the gap is 1 mm.

Therefore, in summation of Figs. 8 and 9, it can be seen that if treatment is provided to plural kinds of tissues by means of application of high-frequency energy (HF) and resistance heat energy (Heat), a high target achievement rate of approximately no less than 90% can be obtained if a gap of no more than 0.7 mm is provided.

Consequently, it can be concluded that: if only high-frequency energy (HF) is used as energy for treatment, the aforementioned fixed distance δ is preferably no more than 0.3 mm; and if high-frequency energy (HF) and resistance heat energy (Heat) are used as energy for treatment, the fixed distance δ is preferably no more than 0.7 mm.

Fig. 10 is a block diagram illustrating mainly an electrical configuration of the medical treatment system.

As illustrated in Fig. 10, the energy source 4 includes a control section 40, a high-frequency energy output circuit (HF energy output circuit) 41, a resistance heater drive circuit 42 and a display section 43. The high-frequency energy output circuit 41, the resistance heater drive circuit 42 and the display section 43 are connected to the control section 40. Also, the foot switch 6 is connected to the control section 40.

Upon the foot switch 6 being turned on, the control section 40 controls the high-frequency energy output circuit (HF energy output circuit) 41 and the resistance heater drive circuit 42 so that treatment via the medical treatment instrument 2 is performed, and upon the foot switch 6 being turned off, controls the high-frequency energy output circuit (HF energy output circuit) 41 and the resistance heater drive circuit 42 to stop the treatment. At the time of the therapy treatment, the control section 40 further controls the display section 43 to display various kinds of display indicating progress of the treatment. Also, the display section 43 has a display function for configuring settings for the control section 40 as well as an operation input function for setting operation. As described above, the control section 40 comprehensively controls the medical treatment system 1.

The high-frequency energy output circuit 41 is electrically connected to the high-frequency electrodes 21 (the first high-frequency electrode 21 a and the second high-frequency electrode 21 b) of the medical treatment instrument 2, and outputs high-frequency energy to the high-frequency electrode 21. The high-frequency energy output circuit 41 is further configured so as to be able to detect an impedance of a circuit based on a signal flowing in the high-frequency energy output circuit 41. Since an impedance relating to the medical treatment system 1 itself is known, an impedance Z of a body tissue grasped between the first high-frequency electrode 21a and the second high-frequency electrode 21b can be calculated based on the detected impedance of the circuit. Therefore, the high-frequency energy output circuit 41 has a sensor function that measures the impedance Z of the body tissue.

The resistance heater drive circuit 42 is electrically connected to the resistance heaters 22 in the medical treatment instrument 2, and outputs electrical energy for resistance heating to the resistance heaters 22. The resistance heater drive circuit 42 further has a sensor function that measures a generated heat temperature T of the resistance heaters 22.

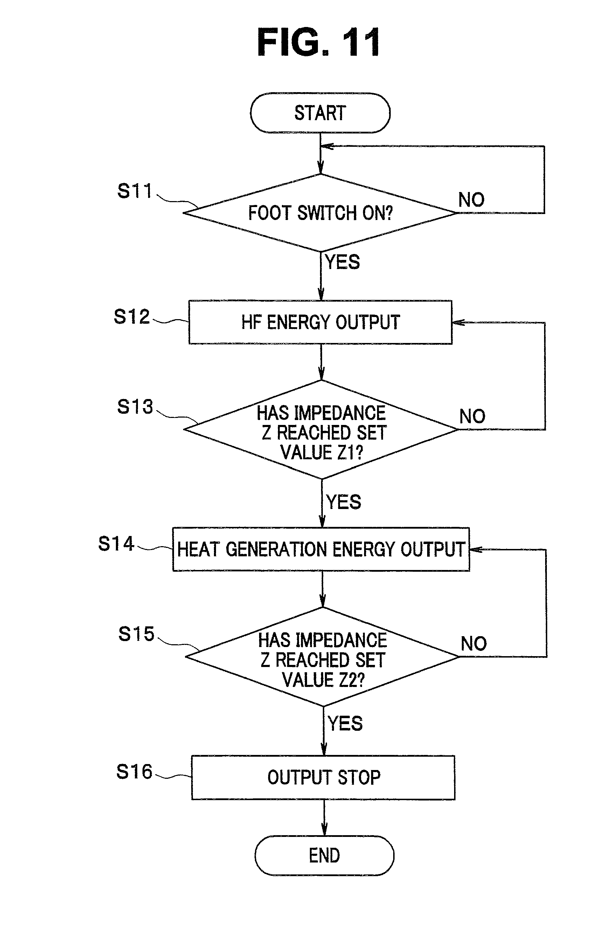

Next, an operation of the medical treatment system 1 according to the present embodiment will be described with reference to Fig. 11. Fig. 11 is a flowchart illustrating processing for performing high-frequency energy and heat energy treatment of a body tissue using the medical treatment system. Also, the operation of the medical treatment system 1 indicates a medical treatment method for applying energy to a body tissue and treating the body tissue.

When a surgeon performs treatment, the surgeon turns on power to the medical treatment system 1, and then operates the display section 43 in the energy source 4 to set, in advance, output conditions for the medical treatment system 1, for example, set power Pset[W] of high-frequency energy output (for a specific example, around 20 [W] to 80 [W]), set temperature Tset[°C] of heat energy output (for a specific example, around 100 [°C] to 300[°C]), and threshold values Z1 and Z2 of an impedance Z of a body tissue (here, Z1 < Z2 and for a specific example, Z1 is around 1000 [Ω] and Z2 is around 2000 [Ω]).

Upon end of the series of settings, the control section 40 of the energy source 4 waits for the foot switch 6 to be turned on (step S11).

Subsequently, as illustrated in Fig. 2, the pinching portion 13 and the shaft 12 of the medical treatment instrument 2 are inserted to, for example, an abdominal cavity through an abdominal wall with the pinching portion 13 closed. Then, the surgeon causes the pinching portion 13 of the medical treatment instrument 2 to face a treatment target body tissue.

Next, in order to hold the treatment target body tissue by the pinching portion 13, the surgeon operates the pinching portion opening/closing lever 14 of the handle 11 to move the sheath 34 to the proximal end portion side of the shaft 12. Upon the sheath 34 being moved to a predetermined position, in the sheath 34, it becomes unable to lock the biasing force of the elastic member 32, and as illustrated in Fig. 3, the second pinching member 24 is opened relative to the first pinching member 23.

Then, the surgeon arranges the treatment target body tissue between the first high-frequency electrode 21a of the first pinching member 23 and the second high-frequency electrode 21b of the second pinching member 24. In this state, the surgeon operates the pinching portion opening/closing lever 14 of the handle 11 to move the sheath 34 to the distal end portion side of the shaft 12. Upon the sheath 34 being moved to the distal end portion side, the sheath 34 closes the base portions 25a and 26a to form a tubular shape against the biasing force of the elastic member 32, that is, as illustrated in Fig. 2, the second pinching member 24 is closed relative to the first pinching member 23. Consequently, the treatment target body tissue is grasped between the first pinching member 23 and the second pinching member 24.

In this phase, the treatment target body tissue is in contact with and held between both the energy application surface of the first high-frequency electrode 21 a and the energy application surface of the second high-frequency electrode 21b, which serve also as the pinching surfaces.

In a state in which the body tissue has been grasped as described above, the surgeon operates the foot switch 6 to be turned on. Then, the control section 40 determines that the foot switch 6 has been turned on in step S 11 described above, and controls the high-frequency energy output circuit 41 to output high-frequency energy of the aforementioned set power Pset[W] to the high-frequency electrode 21 (step S12). Consequently, the high-frequency energy is applied to the body tissue pinched between the first high-frequency electrode 21a and the second high-frequency electrode 21b. Then, Joule heat is generated inside the body tissue, whereby cell membranes are destroyed and intracellular components and extracellular components are homogenized and the body tissue is cauterized. Consequently, the impedance Z of the body tissue increases.

An impedance Z of the grasped body tissue at the time of the high-frequency energy output is measured by the high-frequency energy output circuit 41. The impedance Z at the start of the treatment is, for example, around 60 [Ω]. Then, as high-frequency current is made to flow in the body tissue and the body tissue is thereby cauterized, the cells of the body tissue are dehydrated and the value of the impedance Z increases. Also, as a result of the dehydration of the body tissue, a thickness of the body tissue grasped between the first pinching member 23 and the second pinching member 24 decreases; however, as described above, since the gap maintaining portions 27 and 28 are provided, the distance between the energy application surface of the first high-frequency electrode 21a and the energy application surface of the second high-frequency electrode 21b (distance between the pair of pinching surfaces) is maintained at the fixed distance δ or more.

The control section 40 monitors the impedance Z that is being detected by the high-frequency energy output circuit 41, and determines whether or not the impedance Z becomes the pre-set threshold value Z1 or more (step S13). The threshold value Z1 is set to a value at which the increase in value of the impedance Z becomes slow, which has been found in advance (that is, a value at which a certain degree of dehydration of the body tissue is achieved, which has been found in advance). Then, if the control section 40 determines that the impedance Z is smaller than the threshold value Z1, the control section 40 causes the processing in step S12, that is, the processing for providing high-frequency energy to the body tissue, to be continued.

If the control section 40 determines that the impedance Z has reached the threshold value Z1 or more, the control section 40 controls the resistance heater drive circuit 42 to supply power to the resistance heater 22 so that a temperature of the resistance heater 22 becomes the temperature Tset [°C] set in advance (step S14). Here, the control section 40 controls the power supplied to the resistance heater 22 while monitoring the generated heat temperature T of the resistance heaters 22, which is being measured by the resistance heater drive circuit 42. Consequently, the grasped body tissue is coagulated inward from the surface side of the body tissue that is in close contact with the first high-frequency electrode 21 a, by the heat.

As is well known, water has large specific heat, and thus, before dehydration of a body tissue, even if heat is applied to the body tissue, the temperature gently increases. On the other hand, in the present embodiment, resistance heat energy is applied to a body tissue after the body tissue is dehydrated to a certain degree, enabling a temperature of the body tissue to be effectively increased relative to the amount of applied heat.

Also, even after the coagulation of the body tissue by the heat, the gap maintaining portions 27 and 28 still maintain the distance between the pair of pinching surfaces at the fixed distance δ or more in such a manner as described above.

The control section 40 is continuously monitoring the impedance Z that is being detected by the high-frequency energy output circuit 41, and determines whether or not the impedance Z has reached the pre-set threshold value Z2 or more (step S 15). The threshold value Z2 is set to a value that allows determination of an end of coagulation of a body tissue. Then, if the control section 40 determines that the impedance Z is smaller than the threshold value Z2, the control section 40 causes the processing in step S 14 to be continued.

On the other hand, if the control section 40 determines that the impedance Z has reached the threshold value Z2 or more, the control section 40, for example, while emitting a buzzer sound, causes the high-frequency energy output circuit 41 to stop the output of the high-frequency energy, and causes the resistance heater drive circuit 42 to stop the output of the heat energy (step S16).

Here, body tissues are bonded so as to maintain high pressure resistance performance, without formation of discontinuous parts by the gap maintaining portions 27 and 28, as described above.

Subsequently, in order to remove the treatment target body tissue, the surgeon operates the cutter drive lever 15 to move the cutter 36 in the distal end direction, with the treatment target body tissue grasped by the pair of pinching surfaces.

Consequently, the coagulated body tissue pinched between the pair of pinching surfaces is successively cut off from the proximal end side toward the distal end side by the blade 36a. Upon the movement restricting pin 37 abutting the proximal end side of the long hole 36b, the movement of the cutter 36 toward the distal end side stops. Here, the blade 36a of the cutter 36 reaches the vicinity of the gap maintaining portions 27 and 28. In other words, a body tissue that the gap maintaining portions 27 and 28 abut is positioned on the distal end side the cutter 36 has reached, that is, the distal end side of the cut-off part of the body tissue, and thus no impact is imposed on the bonded parts of the body tissue.

Subsequently, in order to remove the cut-off body tissue from the pinching portion 13, the surgeon operates the pinching portion opening/closing lever 14 to open the pinching portion 13. Consequently, the grasped body tissue comes off from the pinching portion 13.

If there is another treatment target body tissue, the above-described processing is performed in such a manner as described above. Consequently, upon completion of treatment of all treatment target body tissues, the body tissue treatment using the medical treatment system 1 is completed.

The above-described first embodiment provides the following effects.

After cell membranes are destroyed by application of high-frequency energy to a body tissue to increase a heat conductivity of the body tissue, resistance heat energy is applied to the body tissue from the resistance heaters 54 to perform coagulation treatment. Thus, the resistance heat energy application can effectively be performed.

Also, it is known that if electrodes are shorted, output of high-frequency energy via a body tissue becomes impossible, resulting in treatment effect decrease. On the other hand, according to the present embodiment, no shorting of electrodes occurs as a result of provision of the gap maintaining portions 27 and 28, ensuring reliable prevention of treatment effect decrease.

In addition, the gap maintaining portions 27 and 28 are configured so that the distance δ between the energy application surfaces, which serve also as the pair of pinching surfaces, becomes a fixed distance of no more than 0.7 mm (or furthermore, no less than 0.2 mm) as described above, ensuring reliable sealing of plural kinds of body tissues with high pressure resistance performance and a high target achievement rate.

Furthermore, arrangement is made to while monitoring a state of a body tissue grasped by the pinching portion 13 (e.g., the impedance Z of the body tissue and the temperature T of the resistance heaters 22 that provide heat to the body tissue), automatically determine a timing for switching from high-frequency energy supply from heat energy supply based on the threshold value Z1 set in advance and conduct the switching, and automatically determine a timing for terminating the energy supply based on the threshold value Z2 set in advance. Thus, variation of treatment depending on the senses of the surgeons is prevented and treatment suitable for a tissue-denatured state of a body tissue (a cauterized state or a coagulated state) is efficiently and stably performed, enabling homogenization (stabilization) of the tissue.

Consequently, a body tissue can be efficiently treated with smallest possible energy supply loss, without troubling the surgeon, enabling reduction in treatment time, and thus enabling substantial reduction in load on the patient.

Also, the gap maintaining portions 27 and 28 are embedded in the respective cutter guiding grooves 23a and 24a to prevent the gap maintaining portions 27 and 28 from being positioned in the form of an isolated island in the respective energy application surfaces, and thus, no weak part is generated in a sealed body tissue, that is, the pressure resistance performance is not decreased.

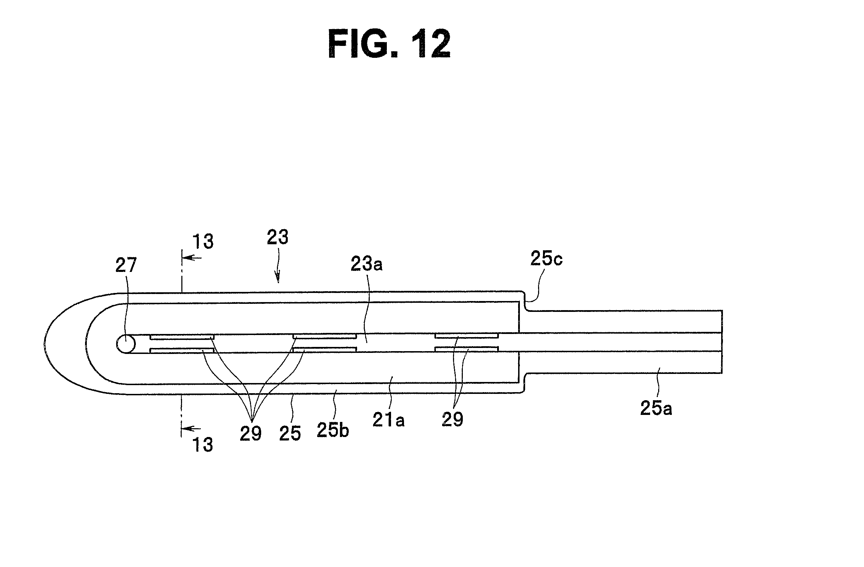

Next, several modifications of the gap maintaining portions provided in the pinching portion will be described with reference to Figs. 12 to 19. Note that for sake of simplicity, the below description is provided in terms of a gap maintaining portion provided on the first pinching member 23 side, a gap maintaining portion similar to the gap maintaining portion is provided also on the second pinching member 24 side.

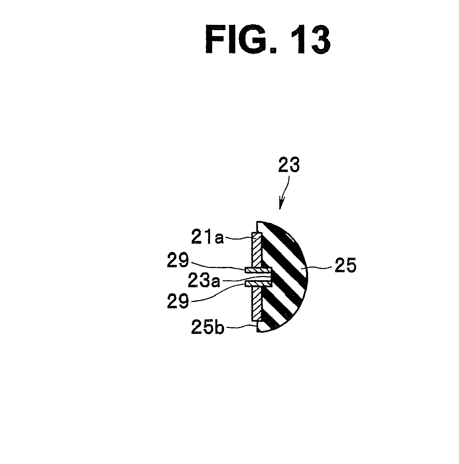

First, a first modification will be described with reference to Figs. 12 and 13. Here, Fig. 12 is a plan view illustrating a first pinching member of a medical treatment instrument according to the first modification, and Fig. 13 is a cross-sectional view of the first pinching member of the medical treatment instrument according to the first modification, along line 13-13 in Fig. 12.

In the first modification, gap maintaining portions 29 are further provided in addition to a gap maintaining portion 27 such as described above.

First, the gap maintaining portion 27 illustrated in Fig. 12 has a configuration that is substantially similar to that of the gap maintaining portion 27 illustrated in, e.g., Fig. 4, but is somewhat different from that of the gap maintaining portion 27 in terms of the gap maintaining portion 27 having a width equal to a groove width of a cutter guiding groove 23a.

Also, the gap maintaining portions 29 are provided in pairs at fixed intervals along an axial direction on opposite inner side surfaces of the cutter guiding groove 23 a. Here, the gap maintaining portions 29 of each pair are provided at a same position in the axial direction so as to face each other. As illustrated in Fig. 13, each gap maintaining portion 29 is provided upright in such a manner that the gap maintaining portion 29 is embedded in the cutter guiding groove 23a and projects from an energy application surface of a first high-frequency electrode 21 a, which serves also as a pinching surface. The gap maintaining portions 29 are also intended to keep a distance δ between energy application surfaces, which serve also as a pair of pinching surfaces, at a fixed distance of no more than 0.7 mm as described above, in cooperation with gap maintaining portions provided on the second pinching member 24 side in a manner similar to the above.

As described above, provision of a gap maintaining portion 27 on the distal end side and a plurality of gap maintaining portions 29 along a cutter guiding groove 23 a enables a distance between a pair of pinching surfaces when a pinching portion is closed to be more stably maintained constant and thus enables the pinching surfaces to be maintained in parallel without inclination.

Also, since each of the gap maintaining portions 27 and 29 is embedded in the cutter guiding groove 23a, no isolated island-like untreated discontinuous parts are formed in a body tissue treated by the first and second high-frequency electrodes 21 a and 21b, enabling provision of treatment with high pressure resistance performance maintained.

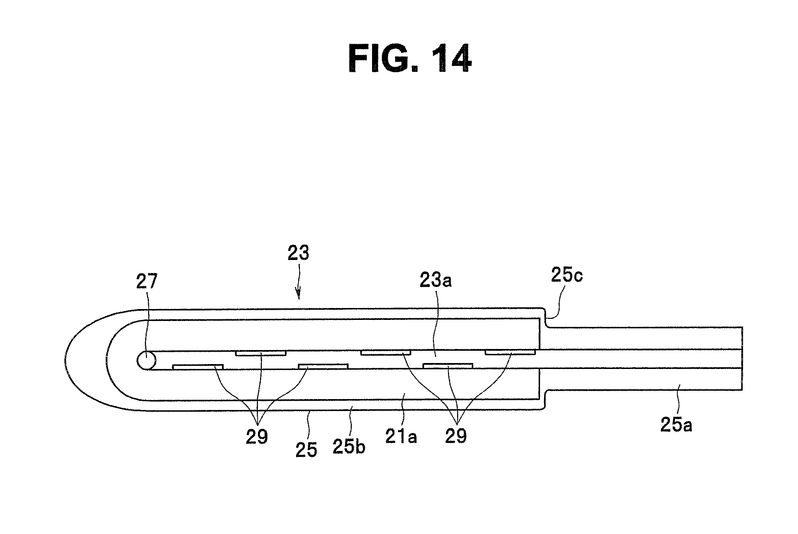

Next, a second modification will be described with reference to Fig. 14. Here, Fig. 14 is a plan view illustrating a first pinching member of a medical treatment instrument according to a second modification.

The second modification is one resulting from arranging the gap maintaining portions 29 provided at fixed intervals alternately at positions that are different in the axial direction in the above-described first modification.

In other words, gap maintaining portions 29 arranged on one inner side surface of a cutter guiding groove 23a and gap maintaining portions 29 arranged on the other inner side surface are alternately arranged with respective positions in the axial direction shifted from one another.

Such second modification enables provision of effects substantially similar to those of the above-described first modification.

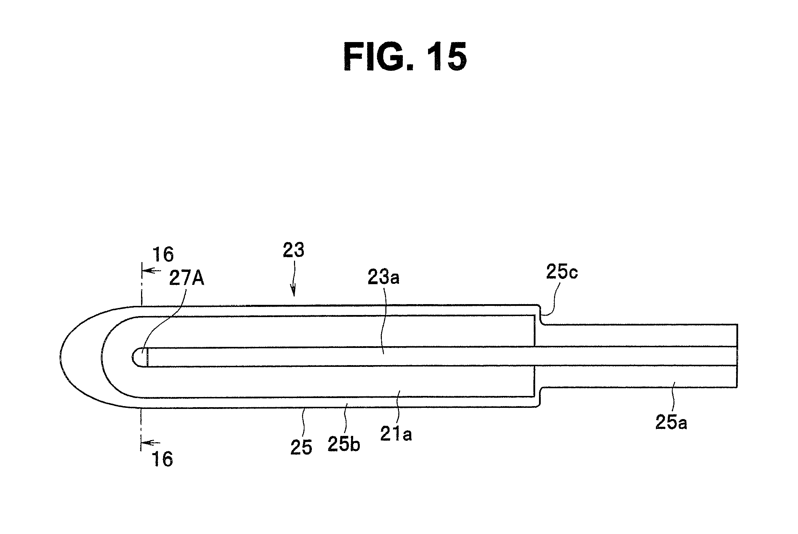

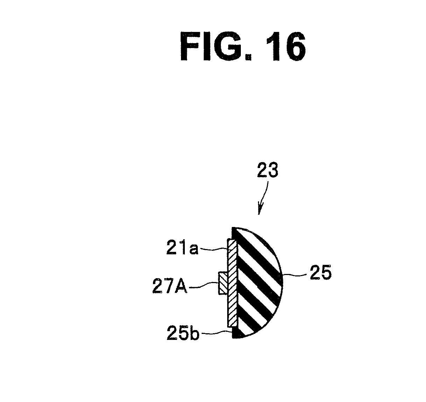

Furthermore, a third modification will be described with reference to Figs. 15 and 16. Here, Fig. 15 is a plan view illustrating a first pinching member of a medical treatment instrument according to a third modification, and Fig. 16 is a cross-sectional view of the first pinching member of the medical treatment instrument according to the third modification, along line 16-16 in Fig. 15.

The third modification is provided with a single gap maintaining portion 27A, but has a configuration that is different from that of the first modification.

In other words, as illustrated in Fig. 16, the gap maintaining portion 27A is formed on an energy application surface of a first high-frequency electrode 21 a, which serves also as a pinching surface, so as to project from the energy application surface. The gap maintaining portion 27A is also intended to keep a distance δ between energy application surfaces, which serve also as a pair of pinching surfaces, at a fixed distance of no more than 0.7 mm as described above, in cooperation with a gap maintaining portion provided on the second pinching member 24 side in a manner similar to the above.

Then, the gap maintaining portion 27A is provided adjacent to a terminal end on the distal end side of the cutter guiding groove 23a so as to face the terminal end in such a manner that the gap maintaining portion 27A is flush with the terminal end. In other words, the configuration is provided so that an end face on the proximal end side of the gap maintaining portion 27A and an end face (inner face) of the terminal end on the distal end side of the cutter guiding groove 23a are flush with each other.

Such configuration also enables treatment to be performed with high pressure resistance performance maintained without formation of untreated isolated island-like discontinuous parts in a body tissue treated by the first and second high-frequency electrodes 21 a and 21b.

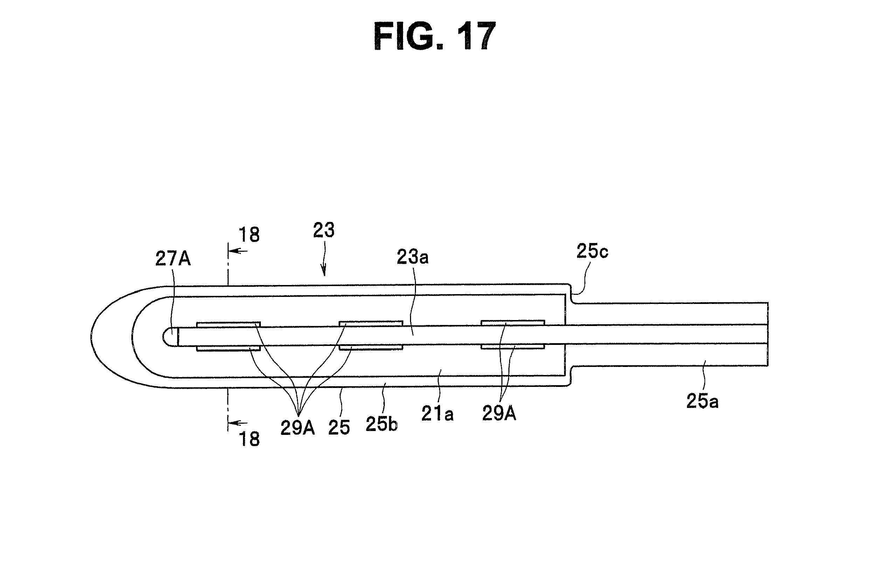

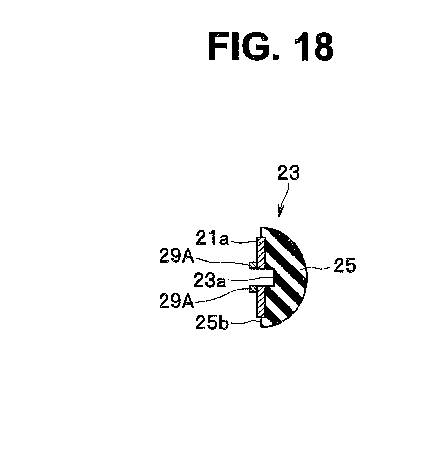

Next, a fourth modification will be described with reference to Figs. 17 and 18. Here, Fig. 17 is a plan view illustrating a first pinching member of a medical treatment instrument according to a fourth modification, and Fig. 18 is a cross-sectional view of the first pinching member of the medical treatment instrument according to the fourth modification, along line 18-18 in Fig. 17.

In the fourth modification, gap maintaining portions 29A are further provided in addition to a gap maintaining portion 27A such as described above.

The gap maintaining portion 29A are provided in pairs at fixed intervals along an axial direction on an energy application surface of a first high-frequency electrode 21 a, which serves also as a pinching surface, provided on both sides of a cutter guiding groove 23a so as to project from the energy application surface. The gap maintaining portions 29A are also intended to keep a distance δ between energy application surfaces, which serve also as a pair of pinching surfaces, at a fixed distance of no more than 0.7 mm as described above, in cooperation with gap maintaining portions provided on the second pinching member 24 side in a manner similar to the above.

Here, the gap maintaining portions 29A of each pair are provided at a same position in the axial direction so as to face each other. As illustrated in Fig. 18, each gap maintaining portion 29A is provided adjacent to an inner side face in such a manner that the gap maintaining portion 29A is flush with the inner side face of the cutter guiding groove 23a. In other words, the configuration is made so that an end face on the cutter guiding groove 23a side of each gap maintaining portion 29A and an end face (inner face) on the first high-frequency electrode 21 a side of the cutter guiding groove 23a are flush with each other.

As described above, provision of a gap maintaining portion 27A on the distal end side and a plurality of gap maintaining portions 29A along a cutter guiding groove 23a enables a distance between a pair of pinching surfaces when a pinching portion is closed to be more stably maintained constant and thus enables the pinching surfaces to be maintained in parallel without inclination.

Also, since each of the gap maintaining portions 27A and 29A is provided adjacent to the cutter guiding groove 23a in such a manner that the gap maintaining portion is flush with the cutter guiding groove 23a (that is, the gap maintaining portion includes a surface that is flush with an inner face of a recess portion of the cutter guiding groove 23a), no isolated island-like untreated discontinuous parts are formed in a body tissue treated by the first and second high-frequency electrodes 21a and 21 b, enabling provision of treatment with high pressure resistance performance maintained.

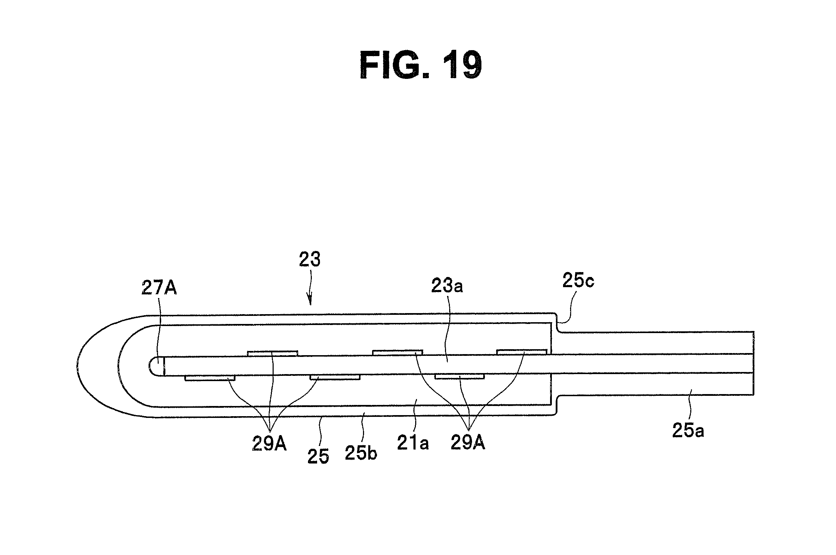

Then, a fifth modification will be described with reference to Fig. 19. Here, Fig. 14 is a plan view illustrating a first pinching member of a medical treatment instrument according to a fifth modification.

The fifth modification is one resulting from arranging gap maintaining portions 29A provided at fixed intervals alternately at positions that are different in the axial direction in the above-described fourth modification.

In other words, gap maintaining portions 29A arranged on one side surface side of the cutter guiding groove 23a and gap maintaining portions 29A arranged on the other side surface are alternately arranged with respective positions in the axial direction shifted from one another.

Such fifth modification enables provision of effects substantially similar to those of the above-described fourth modification.

Furthermore, although in the above description, each of the examples in which a plurality of gap maintaining portions are provided is either a case where a plurality of gap maintaining portions are provided only as gap maintaining portions embedded in a cutter guiding groove or a case where a plurality of gap maintaining portions are provided only as gap maintaining portions adjacent to a cutter guiding groove in such a manner that each gap maintaining portion is flush with the cutter guiding groove, it should be understood that a combination of these cases may be employed.

Also, in Embodiment 1 and the respective modifications, gap maintaining portion(s) are provided on each of a pair of pinching portions; however, the present invention is not limited to this case, and a configuration in which gap maintaining portion(s) are provided only on either one of a pair of pinching portions may be employed.

Furthermore, if the gap maintaining portions 29 or 29A are provided, the gap maintaining portion 27 or 27A is not necessarily needed.

### [Second Embodiment]

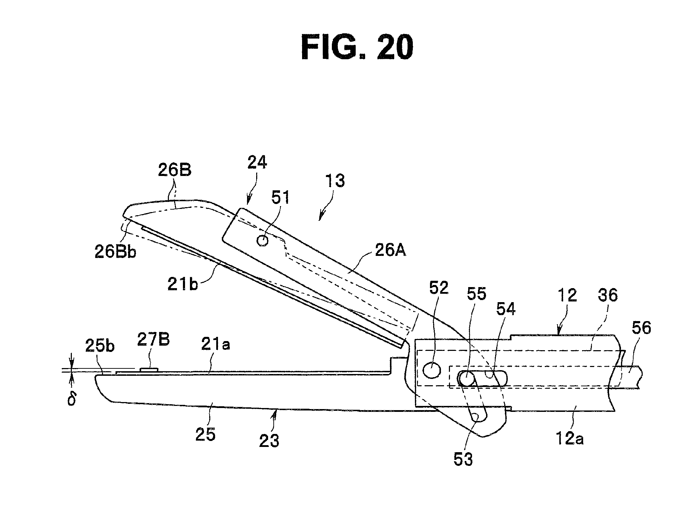

Fig. 20 illustrates a second embodiment of the present invention, and is a side view illustrating a configuration of a pinching portion of a medical treatment instrument configured to be of a seesaw type. In the second embodiment, parts similar to those of the above-described first embodiment are provided with reference numerals that are the same as those of the first embodiment and description thereof will be omitted, and description will be provided mainly on differences.

The present embodiment is an embodiment in which a medical treatment instrument (energy medical treatment instrument) is a linear medical treatment instrument 2 and a seesaw-type jaw is employed.

A pinching portion 13 disposed on the distal end side of a shaft 12 includes a first pinching member 23 and a second pinching member 24.

The first pinching member 23 includes a first pinching member body 25, and the first pinching member body 25 is provided integrally with the shaft 12 so as to extend in an axial direction of the shaft 12.

Also, the second pinching member 24 includes a pinching portion pivot support body 26A on the proximal end side and a second pinching member body 26B on the distal end side, and the proximal end side of the pinching portion pivot support body 26A is pivotally supported so as to be rotatable relative to the shaft 12 and the first pinching member 23 via a pivot shaft 52. The second pinching member body 26B is pivotally supported so as to be swingable relative to the pinching portion pivot support body 26A via a swing shaft 51. In other words, the second pinching member 24 has a structure including the second pinching member body 26B pivotally supported by the swing shaft 51 as a seesaw mechanism in which a pinching surface is brought close to a pinching surface of the first pinching member 23 so as to be parallel with the pinching surface of the first pinching member (a mechanism in which in order to bring the pair of pinching surfaces in parallel with each other when the pair of pinching portions are closed, an energy output section is held so as to be rotatable in a longitudinal direction relative to the relevant pinching portion).

On the proximal end side of the pinching portion pivot support body 26A relative to the pivot shaft 52, a cam hole 53 is formed. On the other hand, in a shaft body 12a of the shaft 12, an axial cam hole 54 is formed. The cam hole 53 and the cam hole 54 are configured so as to intersect with each other, and at a position of the intersection, a cam pin 55 engages with the holes so as to extend through the holes. The cam pin 55 is fixedly attached to the distal end side of an opening/closing drive shaft 56. On the other hand, the proximal end side of the opening/closing drive shaft 56 is mechanistically connected to a pinching portion opening/closing lever 14 (see, e.g., Fig. 1).

With employment of such cam mechanism, if the pinching portion opening/closing lever 14 (see, e.g., Fig. 1) is operated to move the opening/closing drive shaft 56 to the distal end side, the second pinching member 24 is opened relative to the first pinching member 23. On the other hand, if the pinching portion opening/closing lever 14 (see, e.g., Fig. 1) is operated to move the opening/closing drive shaft 56 to the proximal end side, the second pinching member 24 is closed relative to the first pinching member 23.

A first high-frequency electrode 21 a is provided on a holding surface 25b of the first pinching member body 25, and a second high-frequency electrode 21b is provided on a holding surface 26Bb of the second pinching member body 26B. Energy application surfaces of the first high-frequency electrode 21a and the second high-frequency electrode 21 b that face each other serve also as pinching surfaces.

Also, in the present embodiment, a gap maintaining portion is provided only on one of the pair of pinching portions. In other words, a gap maintaining portion 27B formed in the form of a boss by an electrical insulating material is provided so as to project from the surface of the first high-frequency electrode 21a that faces the second high-frequency electrode 21b. The gap maintaining portion 27B is intended to maintain an electrode surface-to-electrode surface distance between the first high-frequency electrode 21 a and the second high-frequency electrode 21b at a fixed distance δ (see Fig. 2) by making the gap maintaining portion 27B abut the surface of the second high-frequency electrode 21b that faces the first high-frequency electrode 21a. Therefore, a height of the projection of the gap maintaining portion 27B from the energy application surface of the first high-frequency electrode 21a is the fixed distance δ. The configuration is made so that the fixed distance δ is, as described above, for example, a distance of no more than 0.7 mm.

In the present embodiment, also, as with the above-described first embodiment, a cutter 36 is provided so as to be movable along the axial direction of the shaft 12, cutter guiding grooves 23a and 24a similar to the above-described ones are provided in the first pinching member body 25 and the second pinching member body 26B, respectively. As with, for example, the one illustrated in Figs. 4 and 5, the gap maintaining portion 27B is provided upright in such a manner that the gap maintaining portion 27B is embedded in a terminal end on the distal end side of the cutter guiding groove 23a.

However, in the present embodiment, also, various modifications similar to the above-described modifications of the first embodiment can be applied. A seesaw-type jaw such as employed in the present embodiment enables a pair of pinching surfaces to be in parallel for a broader range of opening of the pinching portion (that is, a pair of pinching surfaces can be maintained in parallel not only when the pinching portion is closed, but also even when the pinching portion is opened to a certain degree from a closed state). Therefore, in the configuration of the present embodiment, it is preferable to employ a configuration in which a gap maintaining portion is provided at each of a plurality of sites such as illustrated in, for example, Figs. 12, 14, 17 and 19.

Such second embodiment enables provision of effects substantially similar to those of the above-described first embodiment, and also enables a pair of pinching surfaces to be easily brought in parallel without depending on a thickness of a treatment target body tissue because of the medical treatment instrument including a seesaw-type jaw. Also, the second embodiment enables a distance between a pair of pinching surfaces to be more reliably maintained at a fixed distance δ in such medical treatment instrument having high parallelism.

Also, although the above first and second embodiments have been described in terms of an example of a linear medical treatment instrument in which one of a pair of pinching members is fixed to a shaft and the other pinching member is pivotable relative to the shaft, it should be understood that both pinching members may be pivotable relative to the shaft.

### [Third Embodiment]

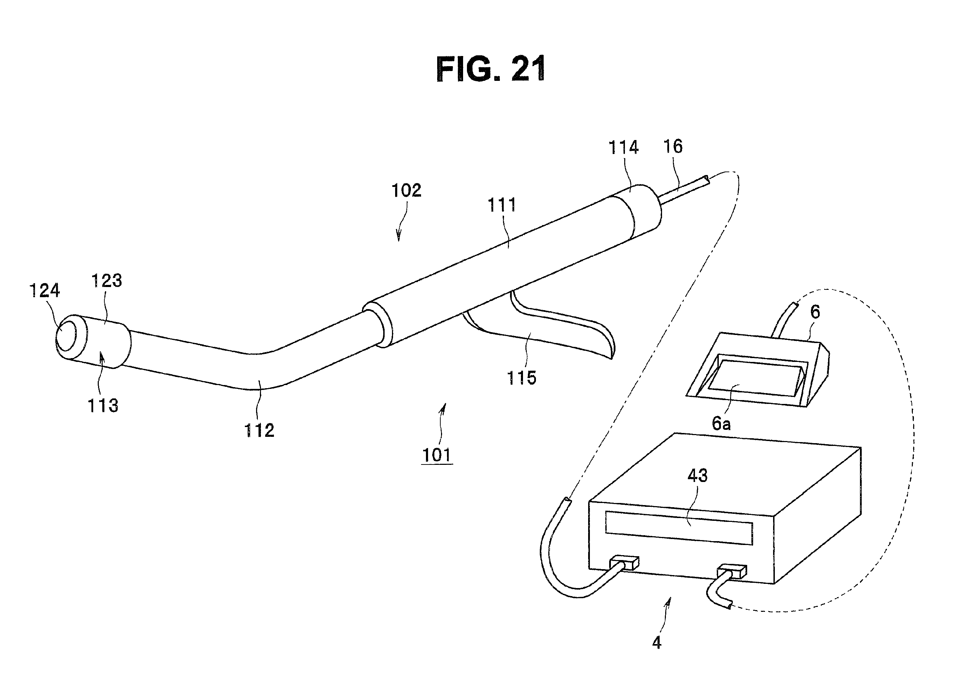

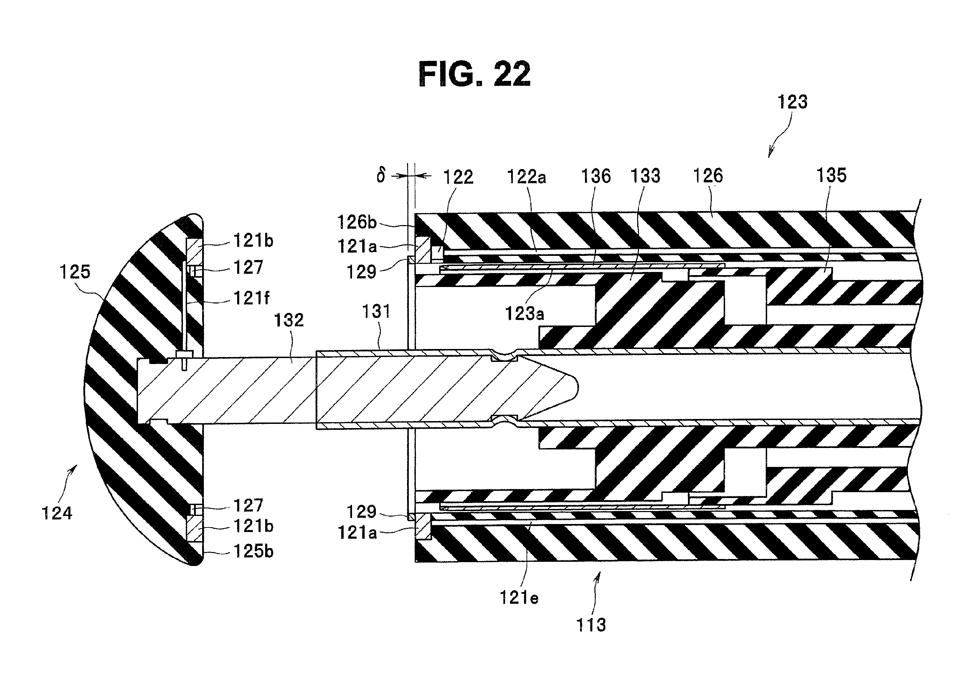

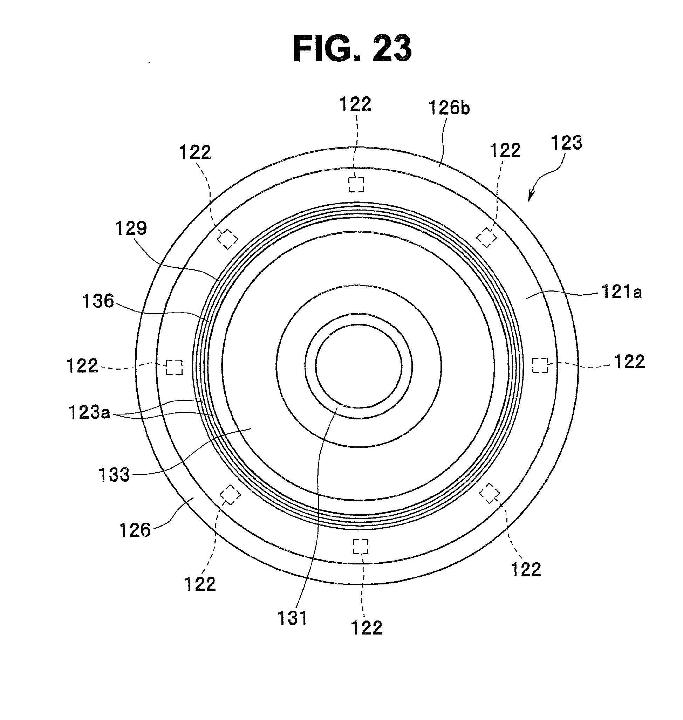

Figs. 21 to 23 illustrate a third embodiment of the present invention: Fig. 21 is a perspective view illustrating a configuration of a medical treatment system including a circular medical treatment instrument; Fig. 22 is a vertical cross-sectional view illustrating a medical treatment instrument when a detachable-side pinching portion is separated from a body-side pinching portion; and Fig. 23 is a plan view illustrating a configuration of a pinching surface of the body-side pinching portion of the medical treatment instrument.

In the third embodiment, parts similar to those of the first and second embodiments are provided with reference numerals that are the same as those of the first and second embodiments and description thereof will be omitted, and description will be provided mainly on differences from the first and second embodiments.

The present embodiment is an embodiment in which a medical treatment instrument (energy medical treatment instrument) is a circular bipolar medical treatment instrument 102 for providing treatment, for example, through or outside an abdominal wall.

As illustrated in Fig. 21, a medical treatment system 101 includes the medical treatment instrument 102, an energy source 4 including a display section 43, and a foot switch 6 including a pedal 6a.

The medical treatment instrument 102 includes a handle 111, a shaft 112 and an openable/closable pinching portion 113. The handle 111 is connected to the energy source 4 via a cable 16. The pinching portion 113 includes a body-side pinching portion 123 and a detachable-side pinching portion 124 configured so as to be brought away from/close to the body-side pinching portion 123.

At the handle 111, a pinching portion opening/closing knob 114, which is a rotatable operation member, and a cutter drive lever 115, which is a swingable operation member, are disposed. Upon the pinching portion opening/closing knob 114 being rotated, for example, clockwise relative to the handle 111, the detachable-side pinching portion 124 of the pinching portion 113 moves away from the body-side pinching portion 123 while a pair of pinching surfaces are maintained in parallel, and upon the pinching portion opening/closing knob 114 being rotated counterclockwise, the detachable-side pinching portion 124 moves close to the body-side pinching portion 123 while the pair of pinching surfaces are maintained in parallel.

The shaft 112 is formed to have an elongated cylindrical shape. In the example illustrated in Fig. 21, in consideration of ease of insertion into a body tissue, the shaft 112 is moderately bent. However, it should be understood that the shaft 112 may be formed linearly.

At a distal end of the shaft 112, the pinching portion 113 is disposed. The pinching portion 113 includes the body-side pinching portion 123, which is a first pinching member formed at the distal end of the shaft 112 and also called first jaw, and the detachable-side pinching portion 124, which is a second pinching member that can be attached/detached to/from the body-side pinching portion 123 and also called second jaw.

As illustrated in Figs. 22 and 23, the body-side pinching portion 123 includes a cylindrical body 126, a frame 133 and an energization pipe 131. The cylindrical body 126 and the frame 133 have electrical insulating properties. The cylindrical body 126 is joined to the distal end of the shaft 112. The frame 133 is disposed on the inner circumferential side of the cylindrical body 126 in such a manner that the frame 133 is fixed to the cylindrical body 126.

The frame 133 has a tubular shape whose center axis part is a communication hole. In the communication hole of the center axis part of the frame 133, the energization pipe 131 is disposed so as to be movable along the center axis of the frame 133 within a predetermined range. The energization pipe 131 is formed in a cylindrical shape by an electrically conductive material, and is connected to the energy source 4 via the shaft 112, the handle 111 and the cable 16. Then, movement of the energization pipe 131 along the center axis of the frame 133 is provided by rotation of the pinching portion opening/closing knob 114.

Between the cylindrical body 126 and the frame 133, a cutter guiding groove (empty space) 123a including a recess portion in an energy application surface is formed. In the cutter guiding groove 123a, a cylindrical cutter 136 is disposed. A proximal end portion of the cutter 136 is fixed to an outer circumferential face of a distal end portion of a cutter pusher 135 disposed inside the shaft 112. A proximal end portion of the cutter pusher 135 is connected to the cutter drive lever 115 of the handle 111 via a non-illustrated mechanism. Thus, upon the cutter drive lever 115 of the handle 111 being operated, the cutter 136 moves and thereby advances/retracts in a direction along the center axis of the frame 133 via the cutter pusher 135.

On the outer circumferential side of the cutter guiding groove 123a at a distal end of the cylindrical body 126, a first high-frequency electrode 121 a and resistance heaters 122 are disposed as an energy output section.

The first high-frequency electrode 121 a is intended to output high-frequency energy, and is a member formed in an annular shape by an electrically conductive material. The energy application surface of the first high-frequency electrode 121a serves also as a pinching surface, and is, for example, flush with (that is, forms a common surface with) a holding surface 126b of the cylindrical body 126. A distal end of a first high-frequency electrode energization line 121 e is fixed to the first high-frequency electrode 121a. The first high-frequency electrode energization line 121e is connected to the energy source 4 via the body-side pinching portion 123, the shaft 112, the handle 111 and the cable 16.

Also, the plurality of resistance heaters 122 are provided and discretely disposed at proper fixed intervals on a circumference of a back face of the first high-frequency electrode 121 a. A distal end of a heater energization line 122a is fixed to each of the resistance heaters 122. The heater energization line 122a is connected to the energy source 4 via the body-side pinching portion 123, the shaft 112, the handle 111 and the cable 16.

At a peripheral edge of the inner circumferential side of the first high-frequency electrode 121 a that faces the cutter guiding groove 123 a, an annular gap maintaining portion 129 is provided to project from the energy application surface of the first high-frequency electrode 121a toward the detachable-side pinching portion 124 side. An inner circumferential face of the gap maintaining portion 129 is flush with (forms a same surface with) the outer circumferential face of the cutter guiding groove 123a (the outer circumferential face is also an inner face of the recess portion of the cutter guiding groove 123a). The gap maintaining portion 129 is intended to maintain an electrode surface-to electrode surface distance between the first high-frequency electrode 121a and a second high-frequency electrode 121b at a fixed distance δ (see Fig. 22) as a result of the gap maintaining portion 129 abutting an energy application surface of the later-described second high-frequency electrode 121b. Therefore, a height of the projection of the gap maintaining portion 27B from the energy application surface of the first high-frequency electrode 121a is the fixed distance δ. As described above, the configuration is made so that the fixed distance δ is a distance of, for example, no more than 0.7 mm.

On the other hand, the detachable-side pinching portion 124 includes an energization shaft 132 having a shaft shape, and a head portion 125 provided on the distal end side of the energization shaft 132.

The energization shaft 132 is formed in a columnar shape by an electrically conductive material, and a distal end portion of the energization shaft 132 is fixed to the head portion 125, and a proximal end portion of the energization shaft 132 is formed in a tapered shape and detachably engages with the energization pipe 131 via a recess and a protrusion. An outer surface of the energization shaft 132 except a part that abuts the energization pipe 131 when the energization shaft 132 is put in the energization pipe 131 is electrically insulated by, e.g., coating.

In the head portion 125, the second high-frequency electrode 121b formed in an annular shape by an electrically conductive material is disposed so as to face the first high-frequency electrode 121a of the body-side pinching portion 123. The energy application surface of the second high-frequency electrode 121b serves also as a pinching surface, and is, for example, flush with (that is, forms a common surface with) a holding surface 125b of the detachable-side pinching portion 124.

An end of the second energization line 121f is fixed to the second high-frequency electrode 121b. Also, the other end of the second energization line 121f is electrically connected to the energization shaft 132. Thus, upon the energization shaft 132 being inserted into the energization pipe 131, the second high-frequency electrode 121b and the energization pipe 131 are electrically connected. Accordingly, the second high-frequency electrode 121b is connected to the energy source 4 via the second energization line 121f, the energization shaft 132, the energization pipe 131, the shaft 112, the handle 111 and the cable 16.

On the inner circumferential side of the second high-frequency electrode 121b disposed in the head portion 125, an annular cutter receiving portion 127 for receiving a blade of the cutter 136 is formed. A receiving surface of the cutter receiving portion 127 is provided on the inner side of the head portion 125 (the side away from the body-side pinching portion 123) relative to the holding surface 125b of the detachable-side pinching portion 124.

Next, an operation of the medical treatment system 101 according to the present embodiment will be described.

With the body-side pinching portion 123 closed relative to the detachable-side pinching portion 124, the pinching portion 113 and the shaft 112 of the medical treatment instrument 102 is inserted into an abdominal cavity through, for example, an abdominal wall. Then, the pinching portion 113 is caused to face a body tissue to be treated.

Next, a surgeon operates the pinching portion opening/closing knob 114 of the handle 111 to rotate the pinching portion opening/closing knob 114, for example, clockwise. Then, the detachable-side pinching portion 124 moves in the distal end direction while the pair of pinching surfaces are maintained in parallel, whereby the detachable-side pinching portion 124 is separated from the body-side pinching portion 123.

Then, the body tissue to be treated is placed between the body-side pinching portion 123 and the detachable-side pinching portion 124 in the opened state. In this state, the surgeon operates the pinching portion opening/closing knob 114 of the handle 111 to rotate the pinching portion opening/closing knob 114, for example, counterclockwise. Consequently, the detachable-side pinching portion 124 moves close to the body-side pinching portion 123 while the pair of pinching surfaces are maintained in parallel, whereby the treatment target body tissue is held between the first high-frequency electrode 121 a of the body-side pinching portion 123 and the second high-frequency electrode 121 b of the detachable-side pinching portion 124.

Here, because of the provision of the gap maintaining portion 129, the electrode surface-to-electrode surface distance between the first high-frequency electrode 121 a and the second high-frequency electrode 121b is maintained at the fixed distance δ or more.

Subsequently, the body tissue is heated by Joule heat provided by subsequent high-frequency energy application and thereby dehydrated, and the body tissue is coagulated by resistance heat energy application, which is similar to the above-described first embodiment.

When the energy application to the body tissue has ended, the body tissue is denatured continuously (in a round annular shape or a rough circular arc shape).

Even in this stage, operation of the gap maintaining portion 129 causes the distance between the electrode surface of the first high-frequency electrode 121 a and the electrode surface of the second high-frequency electrode 121b to be maintained at the fixed distance δ or more.

Subsequently, the surgeon operates the cutter drive lever 115 to move the cutter 136 in the distal end direction. Consequently, the coagulated body tissue is cut off in, e.g., a circular shape or a circular arc shape. When the cut-off body tissue is removed from the pinching portion 113, the surgeon operates the pinching portion opening/closing knob 114 to open the pinching portion 113. Consequently, the grasped body tissue comes off from the pinching portion 113.

Note that although in the above description, only one gap maintaining portion 129 having an annular shape is provided, for example, a plurality of gap maintaining portions each having a boss shape may discretely be arranged at fixed intervals along the cutter guiding groove 123a.

Furthermore, although in the above description, the gap maintaining portion 129 is provided adjacent to the cutter guiding groove 123a so as to be flush with the cutter guiding groove 123a, it should be understood that the gap maintaining portion 129 may be provided so as to be embedded in the cutter guiding groove 123a instead.

Also, although a gap maintaining portion is provided only in the body-side pinching portion, a gap maintaining portion may be provided only in the detachable-side pinching portion or may be provided each of both the body-side pinching portion and the detachable-side pinching portion, which is similar to each of the above described embodiments.

Such third embodiment enables a circular medical treatment instrument to also provide effects substantially similar to those of the above-described first and second embodiments, and enables plural kinds of body tissues to be reliably sealed with high pressure resistance performance and a high target achievement rate without weak parts being generated in the sealed body tissues.

Furthermore, use of a circular medical treatment instrument enables body tissues to be sealed in a rough annular shape.

Note that although each of the above embodiments has been described in terms of an example configuration of a medical treatment instrument including an energy output section on each of a pair of pinching surfaces (that is, a bipolar medical treatment instrument), a configuration including an energy output section only on one of a pair of pinching surfaces (that is, a monopolar medical treatment instrument) may be employed.

Also, the present invention is not limited to the above-described embodiments as they are, and in the practical phase, the present invention can be embodied with any of the elements modified without departing from the spirit of the invention. Also, arbitrary combinations of a plurality of elements disclosed in the embodiments can provide various aspects of the invention. For example, some elements may be deleted from all the elements indicated in the embodiments. Furthermore, elements in different embodiments may arbitrarily be combined. As described above, it should be understood that various modifications and applications are possible without departing from the spirit of the invention.

The present application is filed and claims priority based on Provisional Application No. 61/724,532 filed on November 9, 2012 in the U.S., the disclosed contents of which are incorporated in the specification, claims, and drawings of the present application.

## Claims

1. A medical treatment instrument for applying energy to a body tissue and treating the body tissue, the medical treatment instrument comprising:
a pair of openable/closable pinching portions that pinch the body tissue via a pair of pinching surfaces facing each other;
an energy output section provided in at least one of the pair of pinching portions, the energy output section including an energy application surface for applying energy to the body tissue as at least one of the pair of pinching surfaces;
a cutting member for cutting off the body tissue pinched between the pinching portions, the cutting member being capable of advancing/retracting inside the pinching portions;
a guiding groove including a recess portion in the energy application surface, the guiding groove guiding the cutting member in such a manner that the cutting member can advance/retract inside the pinching portions; and
gap maintaining portions arranged so as to be adjacent to a surface that is flush with an inner face of the recess portion of the guiding groove or be embedded in the guiding groove, the gap maintaining portions projecting between the pair of pinching surfaces facing each other when the pair of pinching portions are closed, to thereby maintain a fixed distance between the pair of pinching surfaces.

2. The medical treatment instrument according to claim 1, wherein in order to maintain a distance between the pair of pinching surfaces facing each other at the fixed distance at one or more sites including a predetermined site, the gap maintaining portions are provided at respective positions corresponding to the one or more sites including the predetermined site in one or both of the pair of pinching surfaces.

3. The medical treatment instrument according to claim 2,
wherein a distal end side of the guiding groove terminates inside the pinching portions; and
wherein the gap maintaining portions are arranged so as to be adjacent to the surface that is flush with the inner face of the recess portion of the guiding groove or be embedded in the guiding groove, with a position corresponding to a terminal end on the distal end side of the guiding groove as the predetermined site.

4. The medical treatment instrument according to claim 3, wherein in order to maintain a distance between the pair of pinching surfaces facing each other at the fixed distance at a plurality of sites, the gap maintaining portions are provided at positions corresponding to the plurality of sites in one or both of the pair of pinching surfaces.

5. The medical treatment instrument according to claim 4, wherein the plurality of gap maintaining portions are provided at a fixed interval along the guiding groove.

6. The medical treatment instrument according to claim 1, wherein the energy output section includes at least one of a high-frequency electrode that generates high-frequency energy, and a resistance heater that generates resistance heat energy.

7. The medical treatment instrument according to claim 1, wherein the gap maintaining portions include at least one material from among a metal processed to have no electrical conductivity, a non-electrically conductive ceramic and a non-electrically conductive resin.

8. The medical treatment instrument according to claim 1, wherein the pair of pinching portions have a shape having a longitudinal direction from a proximal end portion to a distal end portion, the shape allowing the pair of pinching portions to be opened/closed about a pivot shaft, and further includes a mechanism that in order to form the pair of pinching surfaces in parallel when the pair of pinching portions are closed, holds the energy output section in such a manner the energy output section is rotatable in a longitudinal axis direction relative to the pinching portions.

9. The medical treatment instrument according to claim 1, wherein the pair of pinching portions are configured so as to be opened/closed as a result of the pair of pinching portions being brought away from each other or close to each other while the pair of pinching surfaces are maintained in parallel.

10. A medical treatment method for applying energy to a body tissue and treating the body tissue by using a medical treatment instrument including: a pair of openable/closable pinching portions that pinch the body tissue via a pair of pinching surfaces facing each other; an energy output section provided in at least one of the pair of pinching portions, the energy output section including an energy application surface for applying energy to the body tissue as at least one of the pair of pinching surfaces; a cutting member for cutting off the body tissue pinched between the pinching portions, the cutting member being capable of advancing/retracting inside the pinching portions; a guiding groove including a recess portion in the energy application surface, the guiding groove guiding the cutting member in such a manner that the cutting member can advance/retract inside the pinching portions; and gap maintaining portions arranged so as to be adjacent to a surface that is flush with an inner face of the recess portion of the guiding groove or be embedded in the guiding groove, the gap maintaining portions projecting between the pair of pinching surfaces facing each other when the pair of pinching portions are closed, to thereby maintain a fixed distance between the pair of pinching surfaces,
the medical treatment method comprising:
pinching the body tissue via the pair of pinching portions by maintaining a distance between the pair of pinching surfaces to the fixed distance by the gap maintaining portions when the pair of pinching portions are closed;
applying energy to the body tissue from the energy application surface to treat the body tissue while pinching the body tissue via the pair of pinching portions; and
cutting a part of the body tissue adjacent to the gap maintaining portions by advancing the cutting member along the guiding groove.
